(19) Europäisches Patentamt
European Patent Office
Office européen des brevets

(11) Publication number: 0 355 765 A2

# (12) EUROPEAN PATENT APPLICATION

(21) Application number: 89115376.9

(22) Date of filing: 21.08.89

(51) Int. Cl.4: A01N 55/00 , A61L 2/16 , F24F 3/16

(30) Priority: 22.08.88 US 234877

(43) Date of publication of application: 28.02.90 Bulletin 90/09

(84) Designated Contracting States: DE FR GB

(71) Applicant: DOW CORNING CORPORATION
P.O. Box 1767
Midland Michigan 48686-0994(US)

(72) Inventor: White, William Curtis
4409 Bluebird
Midland Michigan(US)

(74) Representative: Spott, Gottfried, Dr. et al
Patentanwälte Spott und Puschmann
Sendlinger-Tor-Platz 11
D-8000 München 2(DE)

## (54) Method of inhibiting the spread of disease and infection in structures.

(57) A method of inhibiting the spread, transmission and incidence of disease, infection and asthma and rhinitis allergies, characteristic of hypersensitivity pneumonitis in structures employing forced or induced air ventilation, heating, humidification and air conditioning systems. Microorganisms contribute to the disease and aggravate the condition of allergies or initiate allergic response. In the method, the level of exposure of occupants of the structure to high levels of microorganisms is reduced by treating interior surfaces of the structure with an organosilicon quaternary ammonium compound. The threshold level of airborne microorganisms is lowered with the organosilicon quaternary ammonium compound in order to prevent reinfestation and the intrinsic antimicrobial activity of the treated surfaces is used to kill transient and resident microbes.

EP 0 355 765 A2

## METHOD OF INHIBITNG THE SPREAD OF DISEASE AND INFECTION IN STRUCTURES

An antimicrobial is an agent that destroys or inhibits the growth of microorganisms. The major classes of microorganisms are bacteria, fungi including mold and mildew, yeasts and algae. Microorganisms can be found in the air, the human body, soil, waste and on all surfaces. The organisms are deposited from the air, food and drink spills, dust, dirt and tracked in soil and from human excreta such as sweat, urine and feces. Organisms grow and multiply when there is available a nutrient source of food such as dirt, organic or inorganic material and living tissue. For growth and multiplication, organisms also require warm temperatures and moisture. When these conditions exist, microorganisms thrive and flourish. Microbial growth, however, leads to many problems such as unpleasant odors ranging from stale to must and mildew-like, to putrid and foul smelling, resembling ammonia. The growths also produce unsightly stains, discoloration and deterioration of many surfaces and materials in which they come into contact. A more serious disadvantage of microbial growth is the production of pathogenic microorganisms, germs, their metabolic products and their somatic and reproductive cell parts, which contribute to the spread of disease and infection. The growth of mold and mildew, for example, significantly aggravates the condition of allergies and airborne bacteria and fungi initiate allergic responses common to many syndromes.

Bound antimicrobials kill organisms on contact and continue to kill organisms without being diffused or leached from the surface. Thus, the bound antimicrobial leaves behind an effective level of active ingredient and is able to control a broad spectrum of microorganisms including gram negative and gram positive bacteria, mold, mildew, fungi, yeast and algae. An exemplary category of bound antimicrobial is an alkoxysilane quaternary ammonium compound and such alkoxysilane quaternary ammonium compounds have been found to be more effective at reducing the number of microorganisms and inhibiting microbially generated odors, than conventional organotin compounds and other organic quaternary ammonium compounds. The silanes of the present invention immobilize on surfaces and bond thereto to provide a coating of immobilized antimicrobial, unlike conventional materials.

In the present invention, this bound characteristic of alkoxysilane quaternary ammonium compounds, as well as their capabilities of performing at effective kill levels beyond prior art types of compositions, is taken advantage of in the treatment of syndromes common to modern energy efficient building, in order to reduce or substantially eliminate the incidence of microorganisms, germs, their metabolic products and their somatic and reproductive cell parts, which contribute to the spread of disease and infection, and to reduce or substantially eliminate the incidence of mold and mildew which aggravate the condition of asthma and rhinitis allergies or initiate such allergic responses.

This invention relates to a method of inhibiting the spread, transmission and incidence of disease, infection and allergies, characteristic of hypersensitivity pneumonitis in structures employing forced or induced air ventilation, heating, humidification and air conditioning systems, in which microorganisms contribute to the spread, transmission and incidence of the disease, infection and aggravate the condition of allergies or initiate allergic response, comprising reducing the level of exposure of occupants of the structure to high levels of microorganisms by treating interior surfaces of the structure with an organosilicon quaternary ammonium compound in an amount effective to significantly destroy, reduce and inhibit the growth and multiplication of bacteria, mildew, mold, fungi, yeast, algae and other pathogenic microorganisms, by the disruption of cell membranes and which cause defacement, surface deterioration, mildew and putrid odors; which also allow for lowering the threshold level of airborne microorganisms, their metabolic products and their somatic and reproductive cell parts, with the organosilicon quaternary ammonium compound in order to prevent reinfestation; and utilizing the intrinsic antimicrobial activity of the treated surfaces to kill transient microbes; the organosilicon quaternary ammonium compound being an organosilane having the general formula selected from the group consisting of:

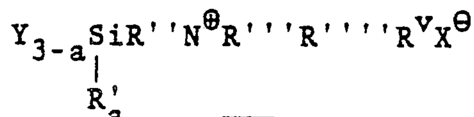

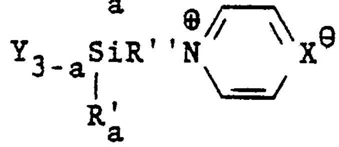

and
wherein, in each formula,
Y is R or RO where R is an alkyl radical of 1 to 4 carbon atoms or hydrogen;
a has a value of 0, 1 or 2;
$R'$ is a methyl or ethyl radical;
$R''$ is an alkylene group of 1 to 4 carbon atoms;
$R'''$, $R''''$ and $R^{v}$ are each independently selected from a group consisting of alkyl radicals of 1 to 18 carbon atoms, $-CH_2C_6H_5$, $-CH_2CH_2OH$, $-CH_2OH$ and $-(CH_2)_xNHC(O)R^{vi}$, wherein x has a value of from 2 to 10 and $R^{vi}$ is a perfluoroalkyl radical having from 1 to 12 carbon atoms;
X is chloride, bromide, fluoride, iodide, acetate or tosylate.

Specifically, the microbes sought to be eliminated herein are those microbes in heating, ventilation and air conditioning systems, humidifiers, cooling towers and other building surfaces and furnishings known to harbor and enhance the lives of the undesirable microbes, which have been linked to hypersensitivity pneumonitis, its related illnesses such as microbially mediated asthma allergies and rhinitis allergies and legionnaires' disease. Among the major microbes are Aspergillus fumigatus, Candida albicans, Penicillium notatum, Sacharomonspora viridis, Thermoactinomyces vulgaris and Thermoactinomyces candidus. There is also included the bacteria Bacillus subtilis; fungal species Alternaria, Coniosporium, Cryptostroma and Cladosporium; other organisms such as Rhizopus, Mucor and Helminthosporium; the species of Legionella pneumophila, Legionella bozemanni and Legionella miadadei; such species as Penicillium frequentans, Penicillium casei and Aspergillus closatus; the various thermophilic and mesophilic actinomycetes including Micropolyspora faeni; and the Thermoactinomyces species T. viridis and T. sacchari; and other antigen producers such as Cephalosporium spp and Aureobasidium pullulans.

The invention also includes the feature of lowering the threshold level of microorganisms by immobilizing on surfaces and bonding thereto a coating of the immobilized antimicrobial organosilicon quaternary ammonium compound in order to prevent reinfestation and the utilization of the intrinsic immobilized antimicrobial activity of the treated surfaces in order to kill transient microbes. This can be assisted by the additional treatment steps of adding the organosilicon quaternary ammonium compound to aqueous medium of the structure employed in conjunction with the forced air ventilation, heating, humidification and air conditioning system of the structure; and passing air from the forced air ventilation, heating, humidification and air conditioning system of the structure through a filter of a flexible, fine-celled, soft, resilient foam formed from a foamable organic system and the organosilane in which the organosilane is used as the co-catalyst, co-surfactant and antimicrobial agent, in producing the foam; as well as passing aqueous medium from the forced air ventilation, heating, humidification and air conditioning system of the structure through such filter; in addition to the treatment of surfaces and furnishings.

It is, therefore, the object of the present invention to inhibit the spread of and the growth of offending antigens responsible for causing hypersensitivity pneumonitis and associated diseases, infections and allergies, common to such disorders; such antigens being somatic reproductive and metabolic product parts of microorganisms.

These and other features, objects and advantages will become apparent upon consideration of the following detailed description of the present invention.

Ammonium compounds in which all of the hydrogen atoms have been substituted by alkyl groups are called quaternary ammonium salts. These compounds may be represented in a general sense by the formula:

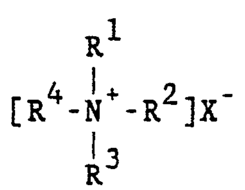

The nitrogen atom includes four covalently bonded substituents that provide a cationic charge. The R groups can be any organic substituent that provides for a carbon and nitrogen bond with similar and dissimilar R groups. The counterion X is typically halogen. Use of quaternary ammonium compounds is based on the lipophilic portion of the molecule which bears a positive charge. Since most surfaces are negatively charged, solutions of these cationic surface active agents are readily absorbed to the negatively charged surface. This affinity for negatively charged surfaces is exhibited by 3-(trimethoxysilyl)-

propyldimethyloctadecyl ammonium chloride (TMS) of the formula:

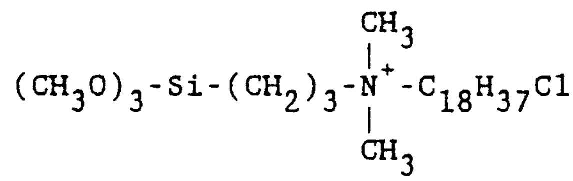

In the presence of moisture, this antimicrobial agent imparts a durable, wash resistant, broad spectrum biostatic surface antimicrobial finish to a substrate. The organosilicon quaternary ammonium compound is leach resistant, nonmigrating and is not consumed by microorganisms. It is effective against gram positive and gram negative bacteria, fungi algae, yeasts, mold, rot, mildew; and malodor and staining caused by these microorganisms. The silicone quaternary ammonium salt provides durable, bacteriostatic, fungistatic and algistatic surfaces. It can be applied to organic or inorganic surfaces as a dilute aqueous or solvent solution of 0.1-1.5 percent by weight of active ingredient. After the alkoxysilane is applied to a surface, it is chemically bonded to the substrate by condensation of the silanol groups at the surface. The compound is a low viscosity, light to dark amber liquid, soluble in water, alcohols, ketones, esters, hydrocarbons and chlorinated hydrocarbons. The compound has been used in applications such as, for example, socks, filtration media, bed sheets, blankets, bedspreads, carpet, draperies, fire hose fabric materials, humidifier belts, mattress pads, mattress ticking, underwear, nonwoven disposable diapers, nonwoven fabrics, outerwear fabrics, nylon hosiery, vinyl paper, wallpaper, polyurethane cushions, roofing materials, sand bags, tents, tarpaulins, sails, rope, athletic and casual shoes, shoe insoles, shower curtains, toilet tanks, toilet seat covers, throw rugs, towels, umbrellas, upholstery fiberfill, intimate apparel, wiping cloths and medical devices.

In the examples as well as in the tables, the antimicrobial composition identified as TMS refers to a product manufactured by the Dow Corning Corporation, Midland, Michigan, as an antimicrobial agent. This compound is 3-(trimethoxysilyl)-propyloctadecyldimethyl ammonium chloride referred to above diluted to forty-two percent active ingredients by weight with methanol.

The silanes useful in this invention have the general formula

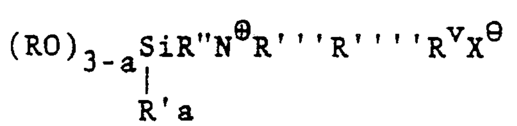 and 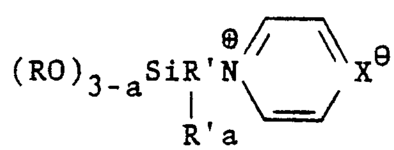

It should be noted that generically, these materials are quaternary ammonium salts of silanes. Most of the silanes falling within the scope of this invention are known silanes and references disclosing such silanes are numerous.

For purposes of this invention, the silanes can be used near or they can be used in solvent or aqueous-solvent solutions. When the silanes are used neat, the inventive process is preferably carried out in a system in which some small amount of water is present. If it is not possible to have a system with some small amount of water present, then a water soluble or water-dispersable, low molecular weight hydrolyzate of the silane may be used. What is important is the fact that the durability of any effect produced by the silane as part of a product requires that the silane molecule react with a surface to a certain extent. The most reactive species, as far as the silanes are concerned, is the ≡SiOH that is formed by hydrolysis of the alkoxy groups present on the silane. The ≡SiOH groups tend to react with the surface and bind the silanes to the surface. It is believed by the inventor that even though the prime mode of coupling to the surface system is by the route described above, it is also believed by the inventor that the alkoxy groups on the silicon atom may also participate in their own right to bind to the surface.

Preferred for this invention is a reactive surface containing some small amount of water. By "reactive", it is meant that the surface must contain some groups which will react with some of the silanols generated by hydrolysis of the silanes of this invention or allow for the association of the ionic quaternary ammonium component.

R in the silanes of this invention are alkyl groups of 1 to 4 carbon atoms. Thus, useful as R in this invention are the methyl, ethyl, propyl and butyl radicals. In the above formulas, RO can also be R. R can also be hydrogen thus indicating the silanol form, i.e. the hydrolyzate. The value of a is 0, 1 or 2 and R′ is a

methyl or ethyl radical.

$R''$ for purposes of this invention is an alkylene group of 1 to 4 carbon atoms. Thus, $R''$ can be alkylene groups such as methylene, ethylene, propylene and butylene. $R'''$, $R''''$ and $R^{v}$ are each independently selected from a group which consists of alkyl radicals or 1 to 18 carbons, $-CH_2C_6H_5$, $-CH_2CH_2OH$, $-CH_2OH$ and $-(CH_2)_xNHC(O)R^{vi}$. x has a value of from 2 to 10 and $R^{vi}$ is a perfluoroalkyl radical having from 1 to 12 carbon atoms. X is chloride, bromide, fluoride, iodide, acetate or tosylate.

Preferred for this invention are the silanes of the general formula

$$(RO)_{3-a}\underset{\displaystyle R'a}{\underset{|}{Si}}R''N^{\oplus}R'''R''''R^{V}X^{\ominus}$$

wherein

R is methyl or ethyl; a has a value of zero; $R''$ is propylene;

$R'''$ is methyl or ethyl; $R''''$ and $R^{v}$ are selected from alkyl groups containing 1 to 18 carbon atoms wherein at least one such group is larger than eight carbon atoms and x is either chloride, acetate or tosylate.

Most preferred for this invention are those silanes having the formula

$(CH_3O)_3Si(CH_2)_3N^{\oplus}(CH_3)_2C_{18}H_{37}Cl^-$ and $(CH_3O)_3Si(CH_2)_3\text{ -}N^{\oplus}CH_3(C_{10}H_{21})_2Cl^-$.

As indicated above, most of these silanes are known from the literature and methods for their preparation are known as well. See, for example, U.S. Patent 4,282,366, issued August 4, 1981; U.S. Patent 4,394,378, issued July 19, 1983 and U.S. Patent 3,661,963 issued May 9, 1972, among others.

Specific silanes within the scope of the invention are represented by the formulae:

$(CH_3O)_3Si(CH_2)_3N^+(CH_3)_2C_{18}H_{37}Cl^-$,
$(CH_3O)_3Si(CH_2)_3N^+(CH_3)_2C_{18}H_{37}Br^-$,
$(CH_3O)_3Si(CH_2)_3N^+(C_{10}H_{21})_2CH_3Cl^-$,
$(CH_3O)_3Si(CH_2)_3N^+(C_{10}H_{21})_2CH_3Br^-$,
$(CH_3O)_3Si(CH_2)_3N^+(CH_3)_3Cl^-$,
$(CH_3O)_3SiCH_2CH_2CH_2P^+(C_6H_5)_3Cl^-$,
$(CH_3O)_3SiCH_2CH_2CH_2P^+(C_6H_5)_3Br^-$,
$(CH_3O)_3SiCH_2CH_2CH_2P^+(CH_3)_3Cl^-$,
$(CH_3O)_3SiCH_2CH_2CH_2P^+(C_6H_{13})_3Cl^-$,
$(CH_3)_3Si(CH_2)_3N^+(CH_3)_2C_{12}H_{25}Cl^-$,
$(CH_3)_3Si(CH_2)_3N^+(C_{10}H_{21})_2CH_3Cl^-$,
$(CH_3)_3Si(CH_2)_3N^+(CH_3)_2C_{18}H_{37}Cl^-$,
$(CH_3O)_3Si(CH_2)_3N^+(CH_3)_2C_4H_9Cl^-$,
$(C_2H_5O)_3Si(CH_2)_3N^+(CH_3)_2C_{18}H_{37}Cl^-$,
$(CH_3O)_3Si(CH_2)_3N^+(CH_3)_2CH_2C_6H_5Cl^-$,
$(CH_3O)_3Si(CH_2)_3N^+(CH_3)_2CH_2CH_2OHCl^-$,

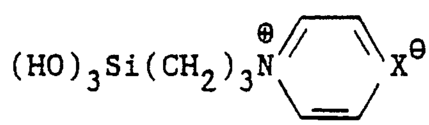

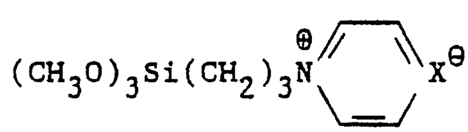

$(CH_3O)_3Si(CH_2)_3N^+(CH_3)_2(CH_2)_3NHC(O)(CF_2)_6CF_3Cl^-$,
$(CH_3O)_3Si(CH_2)_3N^+(C_2H_5)_3Cl^-$.

Hypersensitivity pneumonitis is a granulomatous lung disorder caused by organic dusts inhaled in the workplace. The dusts produce an inflammation in the lung. The disease may also result from and is often incident to working in a building where there is excessive exposure to high levels of microbially contaminated forced or induced air flow from central heating, air conditioning, ventilation and humidification systems. Offending antigens are derived from airborne and surface microorganisms in the contaminated

ventilation system such as antinomycetes, bacteria, fungi, algae and amoebas. Various thermophilic and mesophilic actinomycetes including Micropolyspora faeni and the Thermoactinomyces species T. vulgaris, T. viridis, T. sacchari and T. candidus cause the disease in structures employing contaminated central air conditioning and contaminated humidification systems. Other responsible antigens are Penicillium spp. and Cephalosporium spp.

Hypersensitivity pneumonitis or extrinsic allergic alveolitis represents a group of immunologically induced diseases variously characterized as ventilation pneumonitis, attributed to dusts inhaled from contaminated forced-air systems containing the antigens T. vulgaris and T. candidus; and humidifier fever, humidifier lung or air conditioner lung, characteristic of contaminated humidifiers, dehumidifiers, air conditioning and heating systems and water supplies containing Aureobasidium pullulans. Contaminated water and aerosols in chilled water spray air conditioning systems and humidifiers employing aerosolization are also especially conducive to antigens responsible for hypersensitivity pneumonitis and its related disorders.

Hypersensitivity pneumonitis may not be a reaction to one organism alone but a cumulative reaction to a number of organisms on surfaces and in the air. For example, a spectrum of microorganisms are found in ventilation systems, on ventilation system filters and in the air of the environment. However, the microorganisms generally associated with hypersensitivity pneumonitis are Aspergillus fumigatus, Candida albicans, Penicillium notatum, Sacharomonspora viridis, Thermoactinomyces vulgaris and Thermoactinomyces candidus. Present to a lesser extent are the enzyme of the bacteria Bacillus subtilis; fungal species Aureobasidium, Alternaria, Coniosporium, Cryptostroma and Cladosporium. Other organisms such as Rhizopus, Mucor and Helminthosporium may also be found. Ventilation systems are also associated with illnesses compatible with the diagnosis of legionellosis and, therefore, the species of Legionella pneumophila, Legionella bozemanni and Legionella miadadei are present in such systems. In addition, there should also be mentioned such species as Penicillium frequentans, Penicillium casei and Aspergillus closatus; the various thermophilic and mesophilic actinomycetes including Micropolyspora faeni and the Thermoactinomyces species T. viridis and T. sacchari; and other antigens from species such as Cephalosporium spp and Aureobasidium pullulans.

The following examples relate to a series of tests conducted on carpet samples treated with TMS in order to show the efficacy of this antimicrobial agent against certain test microbes, ranging from bacteria and yeast to fungi. For yeast, Candida albicans were used. The fungi were Aspergillus niger and Trichlophyton mentagrophytes. The seven bacterial microorganisms employed were Staphyloccocus aureus, Streptococcus faecalis, Escherichia coli, Pseudomonas aeruginosa, Klebsiella pneumoniae, Streptococcus mutans and Salmonella typhi. Examples I and II and Tables I-VIII relate to the fungi.

## Example I

In order to demonstrate the effectiveness of TMS against the fungus Trichophyton mentagrophytes, various types of nylon surfaces were treated with the antimicrobial agent and the results are tabulated in Tables I to IV. Comparisons were made on untreated as well as treated surfaces, in order to show the effect of TMS in inhibiting the inactivating test microbes applied to the surfaces. Four types of nylon material surfaces were selected for the tests, including a high-pile cut, a fine velour, a light loop fabric and a heavy-duty loop fabric. Durability of treatment was shown by testing each surface type in its new condition and after 7, 14 and 21, shampoo treatments. For the shampoo treatments, a commercial spray extraction device was used and a non-bacterial shampoo having active groups of nonionic surfactants and phosphates. Each test was repeated three times in order to verify the results obtained.

Test surfaces 50 mm x 50 mm were used as microbial carrier. To prewet the surface, the surface was immersed at 37° C. into a phosphate buffer solution, removed, placed between sterile filter papers in order to remove excess fluid and placed in sterile Petri dishes. Test microbial suspensions were obtained from a nutrient bouillon incubated for 18 hours at 37° C. and stirred at a frequency of 120 rpm by transferring 1 ml of culture bouillon into 9 ml of phosphate buffer. From this 1:10 dilution, a 1:100 dilution was made by placing 1 ml from the first dilution into 9 ml of phosphate buffer. Using the same procedure, a 1:1000 dilution of the suspension was formed. The 1:1000 dilution was used to inoculate the test pieces in sterile Petri dishes by applying 0.01 ml along each lateral edge and diagonally or a total of 0.05 ml of test microbe suspension per microbial carrier. The inoculated pieces were placed into sealed Petri dishes in an air-tight container which was filled to 10% of its volume with water and preheated to 37° C. Incubation of the test pieces was conducted at 37° C. in the container for 4 hours.

The microbial carrier was removed from the container and placed into covered glasses with 200 ml

capacity and filled with 100 ml of Letheen broth and shaken for 10 minutes on a shaking device with a frequency of 180 rpm. Reisolation of the test microbes was carried out by transferring 1 ml from the Letheen broth directly into a Petri Dish followed by one dilution with Letheen broth 1:10 and 1:100. 1 ml of each of the dilutions was placed into a Petri Dish and covered with microbial nutrient Sabouraud Dextrose Agar. The incubation time was 12 weeks. The fungi culture was incubated in a climate-controlled chamber at temperatures of 22 ± 1 °C. and at a relative atmospheric humidity of 96 ± 3%. Readings were conducted during the 1st, 6th and 12th week. A precise quantitative evaluation was impossible because of the biological properties of the fungi. Therefore, the percentage of surface fungal growth on samples in the fungal nutrient dishes was evaluated and is reported in Tables I to IV. It should be apparent from a consideration of Tables I to IV that TMS treatments were not only effectual but durable since even the high number of shampoo treatments had no significant effect on microbial reduction. There was no re-isolation of the fungal species in any test, even after incubating the counting plates for twelve weeks in a climate-controlled chamber. In the tables, the reduction of fungi growth is expressed as growth on a plate in percent of plate surface.

TABLE I

TRICHOPHYTON MENTAGROPHYTES FUNGI GROWTH REDUCTION OF TREATED AND UNTREATED HIGH-PILE CUT NYLON TEST SURFACES

| Shampoo Treatments | Incubation Time (weeks) | Untreated | | | TMS Treated | | |
|---|---|---|---|---|---|---|---|
| | | Run 1 | Run 2 | Run 3 | Run 1 | Run 2 | Run 3 |
| 0 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 7 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 14 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 21 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |

TABLE II

| TRICHOPHYTON MENTAGROPHYTES FUNGI GROWTH REDUCTION OF TREATED AND UNTREATED NYLON FINE VELOUR TEST SURFACES | | | | | | | |
|---|---|---|---|---|---|---|---|
| Shampoo Treatments | Incubation Time (weeks) | Untreated | | | TMS Treated | | |
| | | Run 1 | Run 2 | Run 3 | Run 1 | Run 2 | Run 3 |
| 0 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 7 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 14 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 21 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |

TABLE III

| TRICHOPHYTON MENTAGROPHYTES FUNGI GROWTH REDUCTION OF TREATED AND UNTREATED NYLON LIGHT LOOP FABRIC TEST SURFACES | | | | | | | |
|---|---|---|---|---|---|---|---|
| Shampoo Treatments | Incubation Time (weeks) | Untreated | | | TMS Treated | | |
| | | Run 1 | Run 2 | Run 3 | Run 1 | Run 2 | Run 3 |
| 0 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 7 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 14 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 21 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |

TABLE IV

| TRICHOPHYTON MENTAGROPHYTES FUNGI GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | Incubation Time (weeks) | Untreated | | | TMS Treated | | |
| | | Run 1 | Run 2 | Run 3 | Run 1 | Run 2 | Run 3 |
| 0 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 7 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 14 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 21 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |

Example II

Example I was repeated except that the fungus Aspergillus niger was used as the test microbe. The results are shown in Tables V-VIII.

TABLE V

| ASPERGILLUS NIGER FUNGI GROWTH REDUCTION OF TREATED AND UNTREATED HIGH-PILE CUT NYLON TEST SURFACES | | | | | | | |
|---|---|---|---|---|---|---|---|
| Shampoo Treatments | Incubation Time (weeks) | Untreated | | | TMS Treated | | |
| | | Run 1 | Run 2 | Run 3 | Run 1 | Run 2 | Run 3 |
| 0 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 7 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 14 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 21 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |

TABLE VI

| ASPERGILLUS NIGER FUNGI GROWTH REDUCTION OF TREATED AND UNTREATED NYLON FINE VELOUR TEST SURFACES | | | | | | | |
|---|---|---|---|---|---|---|---|
| Shampoo Treatments | Incubation Time (weeks) | Untreated | | | TMS Treated | | |
| | | Run 1 | Run 2 | Run 3 | Run 1 | Run 2 | Run 3 |
| 0 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 7 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 14 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 21 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |

TABLE VII

| ASPERGILLUS NIGER FUNGI GROWTH REDUCTION OF TREATED AND UNTREATED NYLON LIGHT LOOP FABRIC TEST SURFACES | | | | | | | |
|---|---|---|---|---|---|---|---|
| Shampoo Treatments | Incubation Time (weeks) | Untreated | | | TMS Treated | | |
| | | Run 1 | Run 2 | Run 3 | Run 1 | Run 2 | Run 3 |
| 0 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 7 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 14 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 21 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |

TABLE VIII

| ASPERGILLUS NIGER FUNGI GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | Incubation Time (weeks) | Untreated | | | TMS Treated | | |
| | | Run 1 | Run 2 | Run 3 | Run 1 | Run 2 | Run 3 |
| 0 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 7 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 14 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |
| 21 | 1 | 100 | 100 | 100 | 0 | 0 | 0 |
| | 6 | 100 | 100 | 100 | 0 | 0 | 0 |
| | 12 | 100 | 100 | 100 | 0 | 0 | 0 |

The following example and tables relate to the yeast and bacteria test microbes. The procedure used varied from that set forth in Examples I and II. In Example III, the yeast Candida albicans was the test microbe and the procedure of Example III was repeated for each of the seven bacteria. The results of the tests of Example III on the yeast are reported in Tables 9-16, whereas the seven bacteria are reported in Tables 17-21.

Example III

In order to demonstrate the effectiveness of TMS against the yeast Candida albicans, nylon surfaces were treated with the antimicrobial agent and the results were tabulated in Tables IX the XVI. Comparisons were made on untreated as well as treated surfaces, in order to show the effect of TMS in inhibiting and inactivating test microbes applied to the surfaces. Four types of nylon material surfaces were selected for the tests, including a high-pile cut, a fine velour, a light loop fabric and a heavy-duty loop fabric. Durability of treatment was shown by testing each surface type in its new condition and after 7, 14 and 21 shampoo treatments. For the shampoo treatments, a commercial spray extraction device was used and a non-bacterial shampoo having active groups of nonionic surfactants and phosphates. Each test was repeated three times in order to verify the results obtained.

Test surfaces 50 mm x 50 mm were used as microbe carrier. To prewet the surface, the surface was immersed at 37° C. into a phosphate buffer solution, removed, placed between sterile filter papers in order to remove excess fluid and placed in sterile Petri dishes. Test microbes suspensions were obtained from a nutrient bouillon incubated for 18 hours at 37° C. and stirred at a frequency of 120 rpm by transferring 1 ml of culture bouillon into 9 ml of phosphate buffer. From this 1:10 dilution, a 1:100 dilution was made by placing 1 ml from the first dilution into 9 ml of phosphate buffer. Using the same procedure, a 1:1000 dilution of the suspension was formed. The 1:1000 dilution was used to inoculate the test pieces in sterile Petri dishes by applying 0.01 ml along each lateral edge and diagonally or a total of 0.05 ml of test microbial suspension per microbial carrier. The inoculated pieces were placed into sealed Petri dishes in an air-tight container which was filled to 10% of its volume with water and preheated to 37° C. Incubation of the test pieces was conducted at 37° C. in the container for 4 hours.

The microbial carrier was removed from the container and placed into covered glasses with 200 ml capacity and filled with 100 ml of Letheen broth and shaken for 10 minutes on a shaking device with a frequency of 180 rpm. Reisolation of the test microbes was carried out by transferring 1 ml from the Letheen broth directly into a Petri dish followed by one dilution with Letheen broth 1:10 and 1:100. 1 ml of each of the dilutions was placed into a Petri dish and covered with microbial nutrient agar. The incubation time was 24 hours at 37° C. The grown colony forming units were then counted.

Results are shown in Tables 9-72 in logarithmic figures and each table refers to one piece of carpeting. The dilution stages are included so that a possible total microbial reduction is not expressed as such, but rather defined as "reduction>". In ascertaining reduction, the largest reduction value was selected from the absolute figures of the dilution stages of reisolation, converted into logarithms and subtracted from the microbial seed. In comparing microbial reduction rates of carpeting treated with TMS antimicrobial agent and carpeting not treated, it is noted that untreated carpet reveals a microbial reduction in the range of 1.43 to 3.27 log stages, whereas in the carpeting treated with TMS antimicrobial agent a higher reduction is ascertained. Minute fluctuations of reduction rates in the individual types of carpeting were noted, however, this difference was negligible. Treated carpet is durable since even the high number of shampooing treatments had no significant effect on the microbial reduction rates.

With high pile cut carpet, an above-average inoculation with microbes such as Staphylococcus aureus led to reisolation of test microbes on microbial carriers treated with TMS. The same result was noted for Streptococcus faecalis. In the test microbe species, Salmonella typhi, Streptococcus mutans and Candida albicans, the reduction rates with untreated microbial carriers were low and in microbial carriers treated with TMS antimicrobial agent, the rates reached the limit of detection of the procedure. For fine velour, with the exception of Staphylococcus aureus and Streptococcus faecalis on treated microbial carriers, none of the test microbe species were reisolated. The reduction rates in the microbial carriers of which Staphylococcus aureus was reisolated were above 4 log stages. The microbial reduction rates for Streptococcus faecalis and with treated microbial carriers in which the test microbes was reisolated, were between 2.74 and 3.54 log stages. The treated light loop fabric behaved as the fine velour. Only Staphylococcus aureus was reisolated on treated microbial carriers and a microbial reduction of more than 4 log stages was established. In the reisolation of Streptococcus faecalis of treated microbial carriers, the reduction rates were between 2.74 and 3.47 log stages. Staphylococcus aureus and Streptococcus faecalis were reisolated from the treated heavy-duty loop fabric. The microbial reduction rates for Staphylococcus aureus were between 3.85 and 3.91 log stages and for Streptococcus faecalis, the rates were between 2.85 and 3.77 log stages.

TABLE IX

CANDIDA ALBICANS MICROBIAL GROWTH REDUCTION OF TREATED AND UNTREATED HIGH-PILE CUT NYLON TEST SURFACES

| Shampoo Treatments | Legend | Untreated | | | TMS Treated | | |
|---|---|---|---|---|---|---|---|
| | | Run 1 | Run 2 | Run 3 | Run 1 | Run 2 | Run 3 |
| 0 | A | 5.34 | 5.27 | 5.25 | 5.34 | 5.27 | 5.25 |
| | B | 3.14 | 3.11 | 3.04 | U | U | U |
| | C | 2.10 | 2.16 | 3.21 | >3.34 | >3.27 | >3.25 |
| 7 | A | 5.27 | 5.23 | 5.23 | 5.27 | 5.23 | 5.23 |
| | B | 3.11 | 3.11 | 3.04 | U | U | U |
| | C | 2.16 | 2.12 | 2.19 | >3.27 | >3.23 | >3.23 |
| 14 | A | 5.27 | 5.25 | 5.25 | 5.27 | 5.25 | 5.25 |
| | B | 3.23 | 3.20 | 3.14 | U | U | U |
| | C | 2.04 | 2.05 | 2.11 | >3.27 | >3.25 | >3.25 |
| 21 | A | 5.25 | 5.20 | 5.14 | 5.25 | 5.20 | 5.14 |
| | B | 3.34 | 3.32 | 3.20 | U | U | U |
| | C | 1.91 | 1.88 | 1.94 | >3.25 | >3.20 | >3.14 |

A = Microbial inocculation

B = Reisolation

C = Reduction

U = No microbes reisolatable. Value below limit of detection of two-log stages.

TABLE X

| CANDIDA ALBICANS MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.34 | 5.27 | 5.25 | 5.34 | 5.27 | 5.25 |
| | 10-2 | 14 | 13 | 11 | 0 | 0 | 0 |
| | 10-3 | 3 | 3 | 2 | 0 | 0 | 0 |
| | 10-4 | 0 | 0 | 0 | 0 | 0 | 0 |
| 7 | B | 5.27 | 5.23 | 5.23 | 5.27 | 5.23 | 5.23 |
| | 10-2 | 13 | 13 | 11 | 0 | 0 | 0 |
| | 10-3 | 2 | 2 | 2 | 0 | 0 | 0 |
| | 10-4 | 0 | 0 | 0 | 0 | 0 | 0 |
| 14 | B | 5.27 | 5.25 | 5.25 | 5.27 | 5.25 | 5.25 |
| | 10-2 | 17 | 16 | 14 | 0 | 0 | 0 |
| | 10-3 | 2 | 2 | 2 | 0 | 0 | 0 |
| | 10-4 | 0 | 0 | 0 | 0 | 0 | 0 |
| 21 | B | 5.25 | 5.20 | 5.14 | 5.25 | 5.20 | 5.14 |
| | 10-2 | 22 | 21 | 16 | 0 | 0 | 0 |
| | 10-3 | 3 | 2 | 2 | 0 | 0 | 0 |
| | 10-4 | 0 | 0 | 0 | 0 | 0 | 0 |

A = Microbial inocculation (log). Reisolation per microbial carrier in dilution stage.

B = Microbial inocculation

TABLE XI

CANDIDA ALBICANS MICROBIAL GROWTH REDUCTION OF TREATED AND UNTREATED NYLON FINE VELOUR TEST SURFACES

| Shampoo Treatments | Legend | Untreated | | | TMS Treated | | |
|---|---|---|---|---|---|---|---|
| | | Run 1 | Run 2 | Run 3 | Run 1 | Run 2 | Run 3 |
| 0 | A | 5.34 | 5.27 | 5.25 | 5.34 | 5.27 | 5.25 |
| | B | 2.95 | 2.95 | 2.77 | U | U | U |
| | C | 2.39 | 2.32 | 2.48 | >3.34 | >3.27 | >3.25 |
| 7 | A | 5.27 | 5.23 | 5.23 | 5.27 | 5.23 | 5.23 |
| | B | 3.07 | 3.04 | 3.04 | U | U | U |
| | C | 2.20 | 2.19 | 2.19 | >3.27 | >3.23 | >3.23 |
| 14 | A | 5.27 | 5.25 | 5.25 | 5.27 | 5.25 | 5.25 |
| | B | 3.17 | 3.11 | 3.07 | U | U | U |
| | C | 2.10 | 2.14 | 2.18 | >3.27 | >3.25 | >3.25 |
| 21 | A | 5.25 | 5.20 | 5.14 | 5.25 | 5.20 | 5.14 |
| | B | 3.25 | 3.20 | 3.07 | U | U | U |
| | C | 2.00 | 2.00 | 2.07 | >3.25 | >3.20 | >3.14 |

A = Microbial inocculation

B = Reisolation

C = Reduction

U = No microbes reisolatable. Value below limit of detection of two-log stages.

TABLE XII

| CANDIDA ALBICANS MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.34 | 5.27 | 5.25 | 5.34 | 5.27 | 5.25 |
| | 10-2 | 9 | 9 | 6 | 0 | 0 | 0 |
| | 10-3 | 1 | 0 | 0 | 0 | 0 | 0 |
| | 10-4 | 0 | 0 | 0 | 0 | 0 | 0 |
| 7 | B | 5.27 | 5.23 | 5.23 | 5.27 | 5.23 | 5.23 |
| | 10-2 | 12 | 11 | 11 | 0 | 0 | 0 |
| | 10-3 | 2 | 2 | 1 | 0 | 0 | 0 |
| | 10-4 | 0 | 0 | 0 | 0 | 0 | 0 |
| 14 | B | 5.27 | 5.25 | 5.25 | 5.27 | 5.25 | 5.25 |
| | 10-2 | 15 | 13 | 12 | 0 | 0 | 0 |
| | 10-3 | 2 | 2 | 1 | 0 | 0 | 0 |
| | 10-4 | 0 | 0 | 0 | 0 | 0 | 0 |
| 21 | B | 5.25 | 5.20 | 5.14 | 5.25 | 5.20 | 5.14 |
| | 10-2 | 18 | 16 | 12 | 0 | 0 | 0 |
| | 10-3 | 3 | 2 | 2 | 0 | 0 | 0 |
| | 10-4 | 0 | 0 | 0 | 0 | 0 | 0 |
| A = Microbial inocculation (log). Reisolation per microbial carrier in dilution stage. B = Microbial inocculation | | | | | | | |

TABLE XIII

| CANDIDA ALBICANS MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.34 | 5.27 | 5.25 | 5.34 | 5.27 | 5.25 |
| | B | 2.95 | 2.84 | 2.84 | U | U | U |
| | C | 2.39 | 2.43 | 2.41 | >3.34 | >3.27 | >3.25 |
| 7 | A | 5.27 | 5.23 | 5.23 | 5.27 | 5.23 | 5.23 |
| | B | 3.14 | 3.00 | 2.95 | U | U | U |
| | C | 2.13 | 2.23 | 2.28 | >3.27 | >3.23 | >3.25 |
| 14 | A | 5.27 | 5.25 | 5.25 | 5.27 | 5.25 | 5.25 |
| | B | 3.17 | 3.17 | 3.11 | U | U | U |
| | C | 2.10 | 2.08 | 2.14 | >3.27 | >3.25 | >3.25 |
| 21 | A | 5.25 | 5.20 | 5.14 | 5.25 | 5.20 | 5.14 |
| | B | 3.23 | 3.14 | 3.14 | U | U | U |
| | C | 2.02 | 2.06 | 2.00 | >3.25 | >3.20 | >3.14 |
| A = Microbial inocculation<br>B = Reisolation<br>C = Reduction<br>U = No microbes reisolatable. Value below limit of detection of two-log stages. | | | | | | | |

TABLE XIV

| CANDIDA ALBICANS MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.34 | 5.27 | 5.25 | 5.34 | 5.27 | 5.25 |
| | 10-2 | 9 | 7 | 7 | 0 | 0 | 0 |
| | 10-3 | 1 | 0 | 0 | 0 | 0 | 0 |
| | 10-4 | 0 | 0 | 0 | 0 | 0 | 0 |
| 7 | B | 5.27 | 5.23 | 5.23 | 5.27 | 5.23 | 5.23 |
| | 10-2 | 14 | 10 | 9 | 0 | 0 | 0 |
| | 10-3 | 2 | 1 | 0 | 0 | 0 | 0 |
| | 10-4 | 0 | 0 | 0 | 0 | 0 | 0 |
| 14 | B | 5.27 | 5.25 | 5.25 | 5.27 | 5.25 | 5.25 |
| | 10-2 | 15 | 15 | 13 | 0 | 0 | 0 |
| | 10-3 | 2 | 2 | 3 | 0 | 0 | 0 |
| | 10-4 | 0 | 0 | 0 | 0 | 0 | 0 |
| 21 | B | 5.25 | 5.20 | 5.14 | 5.25 | 5.20 | 5.14 |
| | 10-2 | 17 | 14 | 14 | 0 | 0 | 0 |
| | 10-3 | 2 | 0 | 0 | 0 | 0 | 0 |
| | 10-4 | 0 | 0 | 0 | 0 | 0 | 0 |
| A = Microbial inocculation (log). Reisolation per microbial carrier in dilution stage. B = Microbial inocculation | | | | | | | |

TABLE XV

| CANDIDA ALBICANS MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | Legend | Untreated | | | TMS* Treated | | |
| | | Run 1 | Run 2 | Run 3 | Run 1 | Run 2 | Run 3 |
| 0 | A | 5.34 | 5.27 | 5.25 | 5.34 | 5.27 | 5.25 |
| | B | 3.07 | 3.07 | 3.00 | U | U | U |
| | C | 2.27 | 2.20 | 2.25 | >3.34 | >3.27 | >3.25 |
| 7 | A | 5.27 | 5.23 | 5.23 | 5.27 | 5.23 | 5.23 |
| | B | 3.11 | 3.11 | 3.23 | U | U | U |
| | C | 2.16 | 2.12 | 2.00 | >3.27 | >3.23 | >3.23 |
| 14 | A | 5.27 | 5.25 | 5.25 | 5.27 | 5.25 | 5.25 |
| | B | 3.14 | 3.11 | 3.11 | U | U | U |
| | C | 2.13 | 2.14 | 2.14 | >3.27 | >3.25 | >3.25 |
| 21 | A | 5.25 | 5.20 | 5.14 | 5.25 | 5.20 | 5.14 |
| | B | 3.23 | 3.07 | 3.14 | U | U | U |
| | C | 2.02 | 2.13 | 2.00 | >3.25 | >3.20 | >3.14 |

A = Microbial inocculation
B = Reisolation
C = Reduction
U = No microbes reisolatable. Value below limit of detection of two-log stages.

TABLE XVI

| CANDIDA ALBICANS MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.34 | 5.27 | 5.25 | 5.34 | 5.27 | 5.25 |
| | 10-2 | 12 | 12 | 10 | 0 | 0 | 0 |
| | 10-3 | 2 | 1 | 0 | 0 | 0 | 0 |
| | 10-4 | 0 | 0 | 0 | 0 | 0 | 0 |
| 7 | B | 5.27 | 5.23 | 5.23 | 5.27 | 5.23 | 5.23 |
| | 10-2 | 13 | 13 | 17 | 0 | 0 | 0 |
| | 10-3 | 2 | 2 | 3 | 0 | 0 | 0 |
| | 10-4 | 0 | 0 | 0 | 0 | 0 | 0 |
| 14 | B | 5.27 | 5.25 | 5.25 | 5.27 | 5.25 | 5.25 |
| | 10-2 | 14 | 13 | 13 | 0 | 0 | 0 |
| | 10-3 | 2 | 2 | 2 | 0 | 0 | 0 |
| | 10-4 | 0 | 0 | 0 | 0 | 0 | 0 |
| 21 | B | 5.25 | 5.20 | 5.14 | 5.25 | 5.20 | 5.14 |
| | 10-2 | 17 | 12 | 14 | 0 | 0 | 0 |
| | 10-3 | 2 | 4 | 3 | 0 | 0 | 0 |
| | 10-4 | 0 | 0 | 0 | 0 | 0 | 0 |
| A = Microbial inocculation (log). Reisolation per microbial carrier in dilution stage. B = Microbial inocculation | | | | | | | |

TABLE XVII

| STAPHYLOCOCCUS AUREUS MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 6.47 | 6.32 | 6.25 | 6.47 | 6.32 | 6.25 |
| | B | 4.30 | 4.06 | 3.98 | 2.60 | 2.30 | 2.00 |
| | C | 2.17 | 2.26 | 3.27 | 3.87 | 4.02 | 4.25 |
| 7 | A | 5.91 | 5.86 | 5.76 | 5.91 | 5.86 | 5.76 |
| | B | 4.04 | 4.03 | 3.99 | 2.30 | 2.30 | 2.00 |
| | C | 1.87 | 1.83 | 1.77 | 3.61 | 3.56 | 3.76 |
| 14 | A | 5.66 | 5.59 | 5.62 | 5.66 | 5.59 | 5.62 |
| | B | 3.86 | 3.76 | 3.37 | 2.47 | U | U |
| | C | 1.80 | 1.83 | 1.83 | 3.19 | >3.59 | >3.62 |
| 21 | A | 5.81 | 5.77 | 5.69 | 5.81 | 5.77 | 5.69 |
| | B | 3.99 | 3.93 | 3.91 | U | U | U |
| | C | 1.82 | 1.84 | 1.78 | >3.81 | >3.77 | >3.69 |
| A = Microbial inocculation<br>B = Reisolation<br>C = Reduction<br>U = No microbes reisolatable. Value below limit of detection of two-log stages. | | | | | | | |

TABLE XVIII

| STAPHYLOCOCCUS AUREUS MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 6.47 | 6.32 | 6.25 | 6.47 | 6.32 | 6.25 |
| | 10-2 | 204 | 116 | 96 | 4 | 2 | 1 |
| | 10-3 | 23 | 16 | 9 | 0 | 0 | 0 |
| | 10-4 | 2 | 1 | 1 | 0 | 0 | 0 |
| 7 | B | 5.91 | 5.86 | 5.76 | 5.91 | 5.86 | 5.76 |
| | 10-2 | 110 | 108 | 99 | 2 | 2 | 1 |
| | 10-3 | 12 | 11 | 9 | 0 | 0 | 0 |
| | 10-4 | 2 | 3 | 0 | 0 | 0 | 0 |
| 14 | B | 5.66 | 5.59 | 5.62 | 5.66 | 5.59 | 5.62 |
| | 10-2 | 73 | 58 | 54 | 3 | 0 | 0 |
| | 10-3 | 9 | 7 | 4 | 0 | 0 | 0 |
| | 10-4 | 0 | 0 | 0 | 0 | 0 | 0 |
| 21 | B | 5.81 | 5.77 | 5.69 | 5.81 | 5.77 | 5.69 |
| | 10-2 | 99 | 87 | 82 | 0 | 0 | 0 |
| | 10-3 | 11 | 9 | 10 | 0 | 0 | 0 |
| | 10-4 | 2 | 1 | 2 | 0 | 0 | 0 |
| A = Microbial inocculation (log). Reisolation per microbial carrier in dilution stage.<br>B = Microbial inocculation | | | | | | | |

TABLE XIX

| STAPHYLOCOCCUS AUREUS MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 6.47 | 6.32 | 6.25 | 6.47 | 6.32 | 6.25 |
| | B | 4.27 | 4.21 | 4.19 | 2.30 | 2.00 | 2.00 |
| | C | 2.20 | 2.12 | 2.06 | 4.17 | 4.32 | 4.25 |
| 7 | A | 5.91 | 5.86 | 5.76 | 5.91 | 5.86 | 5.76 |
| | B | 3.99 | 3.72 | 3.71 | U | U | U |
| | C | 1.92 | 2.14 | 2.05 | >3.91 | >3.86 | >3.76 |
| 14 | A | 5.66 | 5.59 | 5.62 | 5.66 | 5.59 | 5.62 |
| | B | 3.66 | 3.55 | 3.64 | U | U | U |
| | C | 2.00 | 2.04 | 1.98 | >3.66 | >3.59 | >3.62 |
| 21 | A | 5.81 | 5.77 | 5.69 | 5.81 | 5.77 | 5.69 |
| | B | 3.97 | 3.91 | 3.80 | U | U | U |
| | C | 1.84 | 1.86 | 1.89 | >3.81 | >3.77 | >3.69 |

A = Microbial inocculation
B = Reisolation
C = Reduction
U = No microbes reisolatable. Value below limit of detection of two-log stages.

TABLE XX

| STAPHYLOCOCCUS AUREUS MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 6.47 | 6.32 | 6.25 | 6.47 | 6.32 | 6.25 |
| | 10-2 | 188 | 163 | 156 | 2 | 1 | 1 |
| | 10-3 | 16 | 14 | 14 | 0 | 0 | 0 |
| | 10-4 | 1 | 1 | 1 | 0 | 0 | 0 |
| 7 | B | 5.91 | 5.86 | 5.76 | 5.91 | 5.86 | 5.76 |
| | 10-2 | 98 | 53 | 52 | 0 | 0 | 0 |
| | 10-3 | 11 | 6 | 7 | 0 | 0 | 0 |
| | 10-4 | 0 | 0 | 0 | 0 | 0 | 0 |
| 14 | B | 5.66 | 5.59 | 5.62 | 5.66 | 5.59 | 5.62 |
| | 10-2 | 46 | 36 | 44 | 0 | 0 | 0 |
| | 10-3 | 3 | 3 | 2 | 0 | 0 | 0 |
| | 10-4 | 0 | 0 | 0 | 0 | 0 | 0 |
| 21 | B | 5.81 | 5.77 | 5.69 | 5.81 | 5.77 | 5.69 |
| | 10-2 | 94 | 83 | 64 | 0 | 0 | 0 |
| | 10-3 | 11 | 7 | 7 | 0 | 0 | 0 |
| | 10-4 | 2 | 0 | 0 | 0 | 0 | 0 |
| A = Microbial inocculation (log). Reisolation per microbial carrier in dilution stage.<br>B = Microbial inocculation | | | | | | | |

TABLE XXI

| STAPHYLOCOCCUS AUREUS MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 6.47 | 6.32 | 6.25 | 6.47 | 6.32 | 6.25 |
| | B | 4.09 | 4.03 | 3.96 | 2.30 | 2.30 | 2.00 |
| | C | 2.38 | 2.29 | 2.29 | 4.17 | 4.17 | 4.25 |
| 7 | A | 5.91 | 5.86 | 5.76 | 5.91 | 5.86 | 5.76 |
| | B | 3.94 | 3.82 | 3.77 | U | U | U |
| | C | 1.97 | 2.04 | 1.99 | >3.91 | >3.86 | >3.76 |
| 14 | A | 5.66 | 5.59 | 5.62 | 5.66 | 5.59 | 5.62 |
| | B | 3.66 | 3.56 | 3.61 | U | U | U |
| | C | 2.00 | 2.03 | 2.01 | >3.66 | >3.59 | >3.62 |
| 21 | A | 5.81 | 5.77 | 5.69 | 5.81 | 5.77 | 5.69 |
| | B | 3.69 | 3.66 | 3.59 | U | U | U |
| | C | 2.12 | 2.11 | 2.10 | >3.81 | >3.77 | >3.69 |
| A = Microbial inocculation<br>B = Reisolation<br>C = Reduction<br>U = No microbes reisolatable. Value below limit of detection of two-log stages. | | | | | | | |

TABLE XXII

| STAPHYLOCOCCUS AUREUS MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 6.47 | 6.32 | 6.25 | 6.47 | 6.32 | 6.25 |
| | 10-2 | 125 | 108 | 93 | 2 | 2 | 1 |
| | 10-3 | 19 | 14 | 12 | 0 | 0 | 0 |
| | 10-4 | 3 | 0 | 2 | 0 | 0 | 0 |
| 7 | B | 5.91 | 5.86 | 5.76 | 5.91 | 5.86 | 5.76 |
| | 10-2 | 88 | 67 | 59 | 0 | 0 | 0 |
| | 10-3 | 12 | 8 | 4 | 0 | 0 | 0 |
| | 10-4 | 2 | 0 | 0 | 0 | 0 | 0 |
| 14 | B | 5.66 | 5.59 | 5.62 | 5.66 | 5.59 | 5.62 |
| | 10-2 | 46 | 37 | 41 | 0 | 0 | 0 |
| | 10-3 | 5 | 2 | 4 | 0 | 0 | 0 |
| | 10-4 | 0 | 0 | 0 | 0 | 0 | 0 |
| 21 | B | 5.81 | 5.77 | 5.69 | 5.81 | 5.77 | 5.69 |
| | 10-2 | 49 | 46 | 39 | 0 | 0 | 0 |
| | 10-3 | 6 | 7 | 4 | 0 | 0 | 0 |
| | 10-4 | 0 | 0 | 0 | 0 | 0 | 0 |

A = Microbial inocculation (log). Reisolation per microbial carrier in dilution stage.

B = Microbial inocculation

TABLE XXIII

STAPHYLOCOCCUS AUREUS MICROBIAL GROWTH REDUCTION OF TREATED AND UNTREATED NYLON HEAVY-DUTY LOOP FABRIC TEST SURFACES

| Shampoo Treatments | Legend | Untreated | | | TMS Treated | | |
|---|---|---|---|---|---|---|---|
| | | Run 1 | Run 2 | Run 3 | Run 1 | Run 2 | Run 3 |
| 0 | A | 6.47 | 6.32 | 6.25 | 6.47 | 6.32 | 6.25 |
| | B | 3.97 | 3.91 | 3.74 | 2.60 | 2.47 | U |
| | C | 2.50 | 2.41 | 2.51 | 3.87 | 3.85 | >4.25 |
| 7 | A | 5.91 | 5.86 | 5.76 | 5.91 | 5.86 | 5.76 |
| | B | 3.98 | 3.96 | 3.64 | 2.00 | 2.00 | U |
| | C | 1.93 | 1.90 | 1.92 | 3.91 | 3.86 | >3.76 |
| 14 | A | 5.66 | 5.59 | 5.62 | 5.66 | 5.59 | 5.62 |
| | B | 3.83 | 3.74 | 3.79 | U | U | U |
| | C | 1.83 | 1.85 | 1.83 | >3.66 | >3.59 | >3.62 |
| 21 | A | 5.81 | 5.77 | 5.69 | 5.81 | 5.77 | 5.69 |
| | B | 3.83 | 3.79 | 3.59 | U | U | U |
| | C | 1.98 | 1.98 | 2.10 | >3.81 | >3.77 | >3.81 |

A = Microbial inocculation
B = Reisolation
C = Reduction
U = No microbes reisolatable. Value below limit of detection of two-log stages.

TABLE XXIV

| STAPHYLOCOCCUS AUREUS MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 6.47 | 6.32 | 6.25 | 6.47 | 6.32 | 6.25 |
| | 10-2 | 94 | 83 | 56 | 4 | 3 | 0 |
| | 10-3 | 11 | 9 | 7 | 0 | 0 | 0 |
| | 10-4 | 2 | 2 | 0 | 0 | 0 | 0 |
| 7 | B | 5.91 | 5.86 | 5.76 | 5.91 | 5.86 | 5.76 |
| | 10-2 | 96 | 93 | 44 | 1 | 1 | 0 |
| | 10-3 | 13 | 11 | 5 | 0 | 0 | 0 |
| | 10-4 | 2 | 1 | 0 | 0 | 0 | 0 |
| 14 | B | 5.66 | 5.59 | 5.62 | 5.66 | 5.59 | 5.62 |
| | 10-2 | 68 | 56 | 62 | 0 | 0 | 0 |
| | 10-3 | 7 | 7 | 8 | 0 | 0 | 0 |
| | 10-4 | 0 | 0 | 0 | 0 | 0 | 0 |
| 21 | B | 5.81 | 5.77 | 5.69 | 5.81 | 5.77 | 5.69 |
| | 10-2 | 69 | 62 | 39 | 0 | 0 | 0 |
| | 10-3 | 9 | 7 | 5 | 0 | 0 | 0 |
| | 10-4 | 1 | 0 | 0 | 0 | 0 | 0 |

A = Microbial inocculation (log). Reisolation per microbial carrier in dilution stage.
B = Microbial inocculation

TABLE XXV

STREPTOCOCCUS FAECALIS MICROBIAL GROWTH REDUCTION OF TREATED AND UNTREATED HIGH-PILE CUT NYLON TEST SURFACES

| Shampoo Treatments | Legend | Untreated | | | TMS Treated | | |
|---|---|---|---|---|---|---|---|
| | | Run 1 | Run 2 | Run 3 | Run 1 | Run 2 | Run 3 |
| 0 | A | 5.91 | 5.83 | 5.77 | 5.91 | 5.83 | 5.77 |
| | B | 4.27 | 4.24 | 4.21 | 2.90 | 3.04 | 3.14 |
| | C | 1.64 | 1.59 | 1.56 | 3.01 | 2.79 | 2.63 |
| 7 | A | 5.89 | 5.82 | 5.74 | 5.89 | 5.82 | 5.74 |
| | B | 4.29 | 4.21 | 4.18 | 3.14 | 3.07 | 2.90 |
| | C | 1.60 | 1.61 | 1.56 | 2.75 | 2.75 | 2.84 |
| 14 | A | 5.87 | 5.83 | 5.77 | 5.87 | 5.83 | 5.77 |
| | B | 4.19 | 4.13 | 4.09 | 2.95 | 2.90 | 2.84 |
| | C | 1.68 | 1.70 | 1.68 | 2.92 | 2.83 | 2.93 |
| 21 | A | 5.69 | 5.64 | 5.62 | 5.69 | 5.64 | 5.62 |
| | B | 4.12 | 4.10 | 4.09 | 2.30 | 2.00 | 2.00 |
| | C | 1.57 | 1.54 | 1.51 | 3.39 | 3.64 | 3.62 |

A = Microbial inocculation
B = Reisolation
C = Reduction
U = No microbes reisolatable. Value below limit of detection of two-log stages.

TABLE XXVI

| STREPTOCOCCUS FAECALIS MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.91 | 5.83 | 5.77 | 5.91 | 5.83 | 5.77 |
| | 10-2 | 189 | 177 | 165 | 8 | 11 | 14 |
| | 10-3 | 21 | 19 | 16 | 0 | 0 | 1 |
| | 10-4 | 4 | 3 | 2 | 0 | 0 | 0 |
| 7 | B | 5.89 | 5.82 | 5.74 | 5.89 | 5.82 | 5.74 |
| | 10-2 | 196 | 163 | 154 | 14 | 12 | 8 |
| | 10-3 | 22 | 18 | 17 | 2 | 1 | 0 |
| | 10-4 | 3 | 2 | 2 | 0 | 0 | 0 |
| 14 | B | 5.87 | 5.83 | 5.77 | 5.87 | 5.83 | 5.77 |
| | 10-2 | 157 | 138 | 124 | 9 | 8 | 7 |
| | 10-3 | 19 | 17 | 11 | 0 | 0 | 0 |
| | 10-4 | 3 | 2 | 1 | 0 | 0 | 0 |
| 21 | B | 5.69 | 5.64 | 5.62 | 5.69 | 5.64 | 5.62 |
| | 10-2 | 134 | 128 | 125 | 2 | 1 | 1 |
| | 10-3 | 14 | 16 | 8 | 0 | 0 | 0 |
| | 10-4 | 3 | 4 | 0 | 0 | 0 | 0 |
| A = Microbial inocculation (log). Reisolation per microbial carrier in dilution stage. B = Microbial inocculation | | | | | | | |

XXVII

| STREPTOCOCCUS FAECALIS MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.91 | 5.83 | 5.77 | 5.91 | 5.83 | 5.77 |
| | B | 4.09 | 4.04 | 3.94 | U | U | U |
| | C | 1.82 | 1.72 | 1.83 | >3.91 | >3.83 | >3.77 |
| 7 | A | 5.89 | 5.82 | 5.74 | 5.89 | 5.82 | 5.74 |
| | B | 4.16 | 4.08 | 4.05 | 2.60 | 2.30 | 2.30 |
| | C | 1.73 | 1.84 | 1.69 | 3.29 | 3.52 | 3.54 |
| 14 | A | 5.87 | 5.83 | 5.77 | 5.87 | 5.83 | 5.77 |
| | B | 4.14 | 4.10 | 4.06 | 2.95 | 2.90 | 2.47 |
| | C | 1.73 | 1.73 | 1.71 | 2.92 | 2.93 | 3.30 |
| 21 | A | 5.69 | 5.64 | 5.62 | 5.69 | 5.64 | 5.62 |
| | B | 4.06 | 4.07 | 4.09 | 2.95 | 2.84 | 2.69 |
| | C | 1.63 | 1.57 | 1.53 | 2.74 | 2.80 | 2.93 |
| A = Microbial inocculation<br>B = Reisolation<br>C = Reduction<br>U = No microbes reisolatable. Value below limit of detection of two-log stages. | | | | | | | |

TABLE XXVIII

| STREPTOCOCCUS FAECALIS MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.91 | 5.83 | 5.77 | 5.91 | 5.83 | 5.77 |
| | 10-2 | 124 | 112 | 88 | 0 | 0 | 0 |
| | 10-3 | 14 | 15 | 10 | 0 | 0 | 0 |
| | 10-4 | 4 | 3 | 2 | 0 | 0 | 0 |
| 7 | B | 5.89 | 5.82 | 5.74 | 5.89 | 5.82 | 5.74 |
| | 10-2 | 146 | 123 | 113 | 4 | 2 | 2 |
| | 10-3 | 17 | 14 | 10 | 0 | 0 | 0 |
| | 10-4 | 2 | 2 | 0 | 0 | 0 | 0 |
| 14 | B | 5.87 | 5.83 | 5.77 | 5.87 | 5.83 | 5.77 |
| | 10-2 | 141 | 128 | 117 | 9 | 8 | 3 |
| | 10-3 | 16 | 13 | 14 | 0 | 0 | 0 |
| | 10-4 | 3 | 3 | 1 | 0 | 0 | 0 |
| 21 | B | 5.69 | 5.64 | 5.62 | 5.69 | 5.64 | 5.62 |
| | 10-2 | 114 | 119 | 124 | 9 | 7 | 5 |
| | 10-3 | 13 | 9 | 14 | 0 | 0 | 0 |
| | 10-4 | 2 | 2 | 1 | 0 | 0 | 0 |

A = Microbial inocculation (log). Reisolation per microbial carrier in dilution stage.

B = Microbial inocculation

TABLE XXIX

| STREPTOCOCCUS FAECALIS MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.91 | 5.83 | 5.77 | 5.91 | 5.83 | 5.77 |
| | B | 4.22 | 4.16 | 4.03 | U | U | U |
| | C | 1.69 | 1.67 | 1.74 | >3.91 | >3.83 | >3.77 |
| 7 | A | 5.89 | 5.82 | 5.74 | 5.89 | 5.82 | 5.74 |
| | B | 4.23 | 4.13 | 4.08 | U | U | U |
| | C | 1.66 | 1.69 | 1.66 | >3.89 | >3.82 | >3.74 |
| 14 | A | 5.87 | 5.83 | 5.77 | 5.87 | 5.83 | 5.77 |
| | B | 4.19 | 4.15 | 4.01 | 2.84 | 2.69 | 2.30 |
| | C | 1.68 | 1.68 | 1.76 | 3.03 | 3.14 | 3.47 |
| 21 | A | 5.69 | 5.64 | 5.62 | 5.69 | 5.64 | 5.62 |
| | B | 4.15 | 4.12 | 4.11 | 2.95 | 2.77 | 2.60 |
| | C | 1.54 | 1.52 | 1.51 | 2.74 | 2.87 | 3.02 |
| A = Microbial inocculation<br>B = Reisolation<br>C = Reduction<br>U = No microbes reisolatable. Value below limit of detection of two-log stages. | | | | | | | |

TABLE XXX

| STREPTOCOCCUS FAECALIS MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.91 | 5.83 | 5.77 | 5.91 | 5.83 | 5.77 |
| | 10-2 | 168 | 146 | 108 | 0 | 0 | 0 |
| | 10-3 | 22 | 19 | 13 | 0 | 0 | 0 |
| | 10-4 | 3 | 2 | 2 | 0 | 0 | 0 |
| 7 | B | 5.89 | 5.82 | 5.74 | 5.89 | 5.82 | 5.74 |
| | 10-2 | 173 | 136 | 122 | 0 | 0 | 0 |
| | 10-3 | 19 | 21 | 14 | 0 | 0 | 0 |
| | 10-4 | 3 | 3 | 2 | 0 | 0 | 0 |
| 14 | B | 5.87 | 5.83 | 5.77 | 5.87 | 5.83 | 5.77 |
| | 10-2 | 158 | 143 | 104 | 7 | 5 | 2 |
| | 10-3 | 23 | 18 | 9 | 0 | 0 | 0 |
| | 10-4 | 4 | 2 | 0 | 0 | 0 | 0 |
| 21 | B | 5.69 | 5.64 | 5.62 | 5.69 | 5.64 | 5.62 |
| | 10-2 | 143 | 132 | 129 | 9 | 6 | 4 |
| | 10-3 | 18 | 16 | 15 | 0 | 0 | 0 |
| | 10-4 | 3 | 2 | 2 | 0 | 0 | 0 |
| A = Microbial inocculation (log). Reisolation per microbial carrier in dilution stage.<br>B = Microbial inocculation | | | | | | | |

TABLE XXXI

| STREPTOCOCCUS FAECALIS MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.91 | 5.83 | 5.77 | 5.91 | 5.83 | 5.77 |
| | B | 4.28 | 4.19 | 4.12 | U | U | U |
| | C | 1.63 | 1.64 | 1.65 | >3.91 | >3.83 | >3.77 |
| 7 | A | 5.89 | 5.82 | 5.74 | 5.89 | 5.82 | 5.74 |
| | B | 4.18 | 4.16 | 4.10 | U | U | U |
| | C | 1.71 | 1.66 | 1.64 | >3.89 | >3.82 | >3.74 |
| 14 | A | 5.87 | 5.83 | 5.77 | 5.87 | 5.83 | 5.77 |
| | B | 4.17 | 4.12 | 4.1 | 2.30 | 2.30 | 2.00 |
| | C | 1.70 | 1.71 | 1.63 | 3.57 | 3.53 | 3.77 |
| 21 | A | 5.69 | 5.64 | 5.62 | 5.69 | 5.64 | 5.62 |
| | B | 4.13 | 4.11 | 4.09 | 2.84 | 2.60 | 2.30 |
| | C | 1.56 | 1.53 | 1.53 | 2.85 | 3.04 | 3.32 |

A = Microbial inocculation

B = Reisolation

C = Reduction

U = No microbes reisolatable. Value below limit of detection of two-log stages.

TABLE XXXII

STREPTOCOCCUS FAECALIS MICROBIAL GROWTH REDUCTION OF TREATED AND UNTREATED NYLON HEAVY-DUTY LOOP FABRIC TEST SURFACES

| Shampoo Treatments | A | Untreated | | | TMS Treated | | |
|---|---|---|---|---|---|---|---|
| | | Run 1 | Run 2 | Run 3 | Run 1 | Run 2 | Run 3 |
| 0 | B | 5.91 | 5.83 | 5.77 | 5.91 | 5.83 | 5.77 |
| | 10-2 | 193 | 156 | 134 | 0 | 0 | 0 |
| | 10-3 | 24 | 19 | 17 | 0 | 0 | 0 |
| | 10-4 | 3 | 3 | 2 | 0 | 0 | 0 |
| 7 | B | 5.89 | 5.82 | 5.74 | 5.89 | 5.82 | 5.74 |
| | 10-2 | 153 | 146 | 127 | 0 | 0 | 0 |
| | 10-3 | 19 | 12 | 12 | 0 | 0 | 0 |
| | 10-4 | 3 | 3 | 2 | 0 | 0 | 0 |
| 14 | B | 5.87 | 5.83 | 5.77 | 5.87 | 5.83 | 5.77 |
| | 10-2 | 148 | 134 | 139 | 2 | 2 | 1 |
| | 10-3 | 23 | 11 | 14 | 0 | 0 | 0 |
| | 10-4 | 3 | 1 | 2 | 0 | 0 | 0 |
| 21 | B | 5.69 | 5.64 | 5.62 | 5.69 | 5.64 | 5.62 |
| | 10-2 | 138 | 129 | 125 | 7 | 4 | 2 |
| | 10-3 | 11 | 9 | 10 | 0 | 0 | 0 |
| | 10-4 | 2 | 0 | 2 | 0 | 0 | 0 |

A = Microbial inocculation (log). Reisolation per microbial carrier in dilution stage.

B = Microbial inocculation

TABLE XXXIII

| ESCHERICHIA COLI MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.93 | 5.86 | 5.83 | 5.93 | 5.86 | 5.83 |
| | B | 4.18 | 4.13 | 4.05 | U | U | U |
| | C | 1.75 | 1.73 | 1.78 | >3.93 | >3.86 | >3.83 |
| 7 | A | 5.78 | 5.77 | 5.63 | 5.78 | 5.77 | 5.63 |
| | B | 4.23 | 4.17 | 4.15 | U | U | U |
| | C | 1.55 | 1.60 | 1.48 | >3.78 | >3.77 | >3.63 |
| 14 | A | 5.82 | 5.80 | 5.78 | 5.82 | 5.80 | 5.78 |
| | B | 4.20 | 4.15 | 4.13 | U | U | U |
| | C | 1.62 | 1.65 | 1.65 | >3.82 | >3.80 | >3.78 |
| 21 | A | 5.82 | 5.78 | 5.77 | 5.82 | 5.78 | 5.77 |
| | B | 3.76 | 3.94 | 3.88 | 2.69 | 2.47 | 2.47 |
| | C | 1.86 | 1.84 | 1.89 | 3.13 | 3.31 | 3.30 |

A = Microbial inocculation

B = Reisolation

C = Reduction

U = No microbes reisolatable. Value below limit of detection of two-log stages.

TABLE XXXIV

| ESCHERICHIA COLI MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.93 | 5.86 | 5.83 | 5.93 | 5.86 | 5.83 |
| | 10-2 | 154 | 138 | 114 | 0 | 0 | 0 |
| | 10-3 | 20 | 16 | 12 | 0 | 0 | 0 |
| | 10-4 | 3 | 2 | 2 | 0 | 0 | 0 |
| 7 | B | 5.78 | 5.77 | 5.63 | 5.78 | 5.77 | 5.63 |
| | 10-2 | 173 | 148 | 143 | 0 | 0 | 0 |
| | 10-3 | 22 | 16 | 14 | 0 | 0 | 0 |
| | 10-4 | 3 | 1 | 2 | 0 | 0 | 0 |
| 14 | B | 5.82 | 5.80 | 5.78 | 5.82 | 5.80 | 5.78 |
| | 10-2 | 162 | 144 | 137 | 0 | 0 | 0 |
| | 10-3 | 23 | 21 | 9 | 0 | 0 | 0 |
| | 10-4 | 5 | 2 | 1 | 0 | 0 | 0 |
| 21 | B | 5.82 | 5.78 | 5.77 | 5.82 | 5.78 | 5.77 |
| | 10-2 | 93 | 88 | 76 | 5 | 3 | 3 |
| | 10-3 | 11 | 9 | 6 | 0 | 0 | 0 |
| | 10-4 | 2 | 2 | 0 | 0 | 0 | 0 |

A = Microbial inocculation (log). Reisolation per microbial carrier in dilution stage.
B = Microbial inocculation

TABLE XXXV

| ESCHERICHIA COLI MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.93 | 5.86 | 5.83 | 5.93 | 5.86 | 5.83 |
| | B | 4.17 | 4.08 | 4.10 | U | U | U |
| | C | 1.76 | 1.78 | 1.73 | >3.93 | >3.86 | >3.83 |
| 7 | A | 5.78 | 5.77 | 5.63 | 5.78 | 5.77 | 5.63 |
| | B | 4.15 | 4.13 | 4.10 | U | U | U |
| | C | 1.65 | 1.64 | 1.53 | >3.78 | >3.77 | >3.63 |
| 14 | A | 5.82 | 5.80 | 5.78 | 5.82 | 5.80 | 5.78 |
| | B | 4.05 | 3.99 | 3.93 | U | U | U |
| | C | 1.77 | 1.81 | 1.85 | >3.82 | >3.80 | >3.78 |
| 21 | A | 5.82 | 5.78 | 5.77 | 5.82 | 5.78 | 5.77 |
| | B | 3.91 | 3.83 | 3.79 | U | U | U |
| | C | 1.91 | 1.95 | 1.98 | >3.82 | >3.78 | >3.77 |

A = Microbial inocculation

B = Reisolation

C = Reduction

U = No microbes reisolatable. Value below limit of detection of two-log stages.

TABLE XXXVI

| ESCHERICHIA COLI MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.93 | 5.86 | 5.83 | 5.93 | 5.86 | 5.83 |
| | 10-2 | 148 | 122 | 126 | 0 | 0 | 0 |
| | 10-3 | 17 | 14 | 11 | 0 | 0 | 0 |
| | 10-4 | 2 | 2 | 1 | 0 | 0 | 0 |
| 7 | B | 5.78 | 5.77 | 5.63 | 5.78 | 5.77 | 5.63 |
| | 10-2 | 144 | 137 | 128 | 0 | 0 | 0 |
| | 10-3 | 16 | 14 | 15 | 0 | 0 | 0 |
| | 10-4 | 3 | 3 | 2 | 0 | 0 | 0 |
| 14 | B | 5.82 | 5.80 | 5.78 | 5.82 | 5.80 | 5.78 |
| | 10-2 | 113 | 98 | 86 | 0 | 0 | 0 |
| | 10-3 | 14 | 11 | 9 | 0 | 0 | 0 |
| | 10-4 | 2 | 2 | 0 | 0 | 0 | 0 |
| 21 | B | 5.82 | 5.78 | 5.77 | 5.82 | 5.78 | 5.77 |
| | 10-2 | 82 | 68 | 63 | 0 | 0 | 0 |
| | 10-3 | 10 | 8 | 7 | 0 | 0 | 0 |
| | 10-4 | 2 | 0 | 0 | 0 | 0 | 0 |
| A = Microbial inocculation (log). Reisolation per microbial carrier in dilution stage.<br>B = Microbial inocculation | | | | | | | |

TABLE XXXVII

| ESCHERICHIA COLI MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.93 | 5.86 | 5.83 | 5.93 | 5.86 | 5.83 |
| | B | 4.14 | 4.12 | 4.10 | U | U | U |
| | C | 1.79 | 1.74 | 1.73 | >3.93 | >3.86 | >3.83 |
| 7 | A | 5.78 | 5.77 | 5.63 | 5.78 | 5.77 | 5.63 |
| | B | 4.16 | 4.10 | 4.05 | U | U | U |
| | C | 1.72 | 1.67 | 1.58 | >3.78 | >3.77 | >3.63 |
| 14 | A | 5.82 | 5.80 | 5.78 | 5.82 | 5.80 | 5.78 |
| | B | 4.04 | 3.98 | 3.97 | U | U | U |
| | C | 1.78 | 1.82 | 1.81 | >3.82 | >3.80 | >3.78 |
| 21 | A | 5.82 | 5.78 | 5.77 | 5.82 | 5.78 | 5.77 |
| | B | 3.93 | 3.91 | 3.85 | U | U | U |
| | C | 1.89 | 1.87 | 1.92 | >3.82 | >3.78 | >3.77 |

A = Microbial inocculation
B = Reisolation
C = Reduction
U = No microbes reisolatable. Value below limit of detection of two-log stages.

TABLE XXXVIII

| ESCHERICHIA COLI MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.93 | 5.86 | 5.83 | 5.93 | 5.86 | 5.83 |
| | 10-2 | 139 | 134 | 126 | 0 | 0 | 0 |
| | 10-3 | 17 | 18 | 12 | 0 | 0 | 0 |
| | 10-4 | 3 | 4 | 2 | 0 | 0 | 0 |
| 7 | B | 5.78 | 5.77 | 5.63 | 5.78 | 5.77 | 5.63 |
| | 10-2 | 146 | 127 | 114 | 0 | 0 | 0 |
| | 10-3 | 22 | 18 | 13 | 0 | 0 | 0 |
| | 10-4 | 4 | 3 | 2 | 0 | 0 | 0 |
| 14 | B | 5.82 | 5.80 | 5.78 | 5.82 | 5.80 | 5.78 |
| | 10-2 | 111 | 97 | 94 | 0 | 0 | 0 |
| | 10-3 | 14 | 11 | 8 | 0 | 0 | 0 |
| | 10-4 | 2 | 2 | 0 | 0 | 0 | 0 |
| 21 | B | 5.82 | 5.78 | 5.77 | 5.82 | 5.78 | 5.77 |
| | 10-2 | 87 | 83 | 72 | 0 | 0 | 0 |
| | 10-3 | 10 | 11 | 9 | 0 | 0 | 0 |
| | 10-4 | 2 | 2 | 1 | 0 | 0 | 0 |
| A = Microbial inocculation (log). Reisolation per microbial carrier in dilution stage.<br>B = Microbial inocculation | | | | | | | |

TABLE XXXIX

| ESCHERICHIA COLI MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.93 | 5.86 | 5.83 | 5.93 | 5.86 | 5.83 |
| | B | 4.17 | 4.14 | 4.08 | U | U | U |
| | C | 1.76 | 1.72 | 1.75 | >3.93 | >3.86 | >3.83 |
| 7 | A | 5.78 | 5.77 | 5.63 | 5.78 | 5.77 | 5.63 |
| | B | 4.12 | 4.09 | 4.08 | U | U | U |
| | C | 1.66 | 1.68 | 1.55 | >3.78 | >3.77 | >3.63 |
| 14 | A | 5.82 | 5.80 | 5.78 | 5.82 | 5.80 | 5.78 |
| | B | 3.99 | 3.92 | 3.87 | U | U | U |
| | C | 1.83 | 1.88 | 1.91 | >3.82 | >3.80 | >3.78 |
| 21 | A | 5.82 | 5.78 | 5.77 | 5.82 | 5.78 | 5.77 |
| | B | 3.86 | 3.86 | 3.85 | U | U | U |
| | C | 1.96 | 1.92 | 1.92 | >3.82 | >3.78 | >3.77 |

A = Microbial inocculation
B = Reisolation
C = Reduction
U = No microbes reisolatable. Value below limit of detection of two-log stages.

TABLE XL

| ESCHERICHIA COLI MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.93 | 5.86 | 5.83 | 5.93 | 5.86 | 5.83 |
| | 10-2 | 149 | 141 | 121 | 0 | 0 | 0 |
| | 10-3 | 19 | 14 | 17 | 0 | 0 | 0 |
| | 10-4 | 3 | 2 | 3 | 0 | 0 | 0 |
| 7 | B | 5.78 | 5.77 | 5.63 | 5.78 | 5.77 | 5.63 |
| | 10-2 | 134 | 125 | 122 | 0 | 0 | 0 |
| | 10-3 | 18 | 16 | 11 | 0 | 0 | 0 |
| | 10-4 | 3 | 3 | 3 | 0 | 0 | 0 |
| 14 | B | 5.82 | 5.80 | 5.78 | 5.82 | 5.80 | 5.78 |
| | 10-2 | 98 | 85 | 75 | 0 | 0 | 0 |
| | 10-3 | 12 | 9 | 6 | 0 | 0 | 0 |
| | 10-4 | 1 | 2 | 0 | 0 | 0 | 0 |
| 21 | B | 5.82 | 5.78 | 5.77 | 5.82 | 5.78 | 5.77 |
| | 10-2 | 74 | 73 | 71 | 0 | 0 | 0 |
| | 10-3 | 8 | 9 | 6 | 0 | 0 | 0 |
| | 10-4 | 0 | 0 | 0 | 0 | 0 | 0 |
| A = Microbial inocculation (log). Reisolation per microbial carrier in dilution stage. B = Microbial inocculation | | | | | | | |

TABLE XLI

| PSEUDOMONAS AERUGINOSA MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.83 | 5.72 | 5.70 | 5.83 | 5.72 | 5.70 |
| | B | 4.24 | 4.18 | 4.15 | 2.60 | 2.30 | U |
| | C | 1.59 | 1.54 | 1.55 | 3.23 | 3.42 | >3.70 |
| 7 | A | 5.65 | 5.64 | 5.59 | 5.65 | 5.64 | 5.59 |
| | B | 4.15 | 4.12 | 4.10 | U | U | U |
| | C | 1.50 | 1.52 | 1.49 | >3.65 | >3.64 | >3.59 |
| 14 | A | 5.55 | 5.54 | 5.51 | 5.55 | 5.54 | 5.51 |
| | B | 4.05 | 3.99 | 3.95 | U | U | U |
| | C | 1.50 | 1.55 | 1.56 | >3.55 | >3.54 | >3.51 |
| 21 | A | 5.57 | 5.55 | 5.50 | 5.57 | 5.55 | 5.50 |
| | B | 4.10 | 4.09 | 4.07 | 2.30 | 2.30 | 2.00 |
| | C | 1.57 | 1.56 | 1.43 | 3.27 | 3.25 | 3.50 |

A = Microbial inocculation
B = Reisolation
C = Reduction
U = No microbes reisolatable. Value below limit of detection of two-log stages.

TABLE XLII

| PSEUDOMONAS AERUGINOSA MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.83 | 5.72 | 5.70 | 5.83 | 5.72 | 5.70 |
| | 10-2 | 167 | 152 | 144 | 4 | 2 | 0 |
| | 10-3 | 23 | 19 | 16 | 0 | 0 | 0 |
| | 10-4 | 4 | 3 | 1 | 0 | 0 | 0 |
| 7 | B | 5.65 | 5.64 | 5.59 | 5.65 | 5.64 | 5.59 |
| | 10-2 | 143 | 134 | 127 | 0 | 0 | 0 |
| | 10-3 | 16 | 17 | 14 | 0 | 0 | 0 |
| | 10-4 | 2 | 2 | 2 | 0 | 0 | 0 |
| 14 | B | 5.55 | 5.54 | 5.51 | 5.55 | 5.54 | 5.51 |
| | 10-2 | 113 | 98 | 90 | 0 | 0 | 0 |
| | 10-3 | 14 | 11 | 8 | 0 | 0 | 0 |
| | 10-4 | 2 | 1 | 0 | 0 | 0 | 0 |
| 21 | B | 5.57 | 5.55 | 5.50 | 5.57 | 5.55 | 5.50 |
| | 10-2 | 126 | 124 | 118 | 0 | 0 | 0 |
| | 10-3 | 14 | 14 | 16 | 0 | 0 | 0 |
| | 10-4 | 4 | 3 | 1 | 0 | 0 | 0 |
| A = Microbial inocculation (log). Reisolation per microbial carrier in dilution stage.<br>B = Microbial inocculation | | | | | | | |

TABLE XLIII

| PSEUDOMONAS AERUGINOSA MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.83 | 5.72 | 5.70 | 5.83 | 5.72 | 5.70 |
| | B | 4.16 | 4.11 | 4.06 | U | U | U |
| | C | 1.67 | 1.61 | 1.64 | >3.83 | >3.72 | >3.70 |
| 7 | A | 5.65 | 5.64 | 5.59 | 5.65 | 5.64 | 5.59 |
| | B | 3.96 | 3.93 | 3.86 | U | U | U |
| | C | 1.69 | 1.71 | 1.73 | >3.65 | >3.64 | >3.59 |
| 14 | A | 5.55 | 5.54 | 5.51 | 5.55 | 5.54 | 5.51 |
| | B | 3.82 | 3.81 | 3.83 | U | U | U |
| | C | 1.73 | 1.73 | 1.68 | >3.55 | >3.54 | >3.51 |
| 21 | A | 5.57 | 5.55 | 5.50 | 5.57 | 5.55 | 5.50 |
| | B | 3.88 | 3.86 | 3.90 | U | U | U |
| | C | 1.69 | 1.69 | 1.60 | >3.57 | >3.55 | >3.50 |

A = Microbial inocculation
B = Reisolation
C = Reduction
U = No microbes reisolatable. Value below limit of detection of two-log stages.

TABLE XLIV

| PSEUDOMONAS AERUGINOSA MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.83 | 5.72 | 5.70 | 5.83 | 5.72 | 5.70 |
| | 10-2 | 146 | 129 | 116 | 0 | 0 | 0 |
| | 10-3 | 17 | 12 | 14 | 0 | 0 | 0 |
| | 10-4 | 2 | 2 | 1 | 0 | 0 | 0 |
| 7 | B | 5.65 | 5.64 | 5.59 | 5.65 | 5.64 | 5.59 |
| | 10-2 | 92 | 87 | 74 | 0 | 0 | 0 |
| | 10-3 | 10 | 10 | 8 | 0 | 0 | 0 |
| | 10-4 | 2 | 1 | 0 | 0 | 0 | 0 |
| 14 | B | 5.55 | 5.54 | 5.51 | 5.55 | 5.54 | 5.51 |
| | 10-2 | 67 | 65 | 68 | 0 | 0 | 0 |
| | 10-3 | 9 | 8 | 8 | 0 | 0 | 0 |
| | 10-4 | 0 | 0 | 0 | 0 | 0 | 0 |
| 21 | B | 5.57 | 5.55 | 5.50 | 5.57 | 5.55 | 5.50 |
| | 10-2 | 77 | 73 | 81 | 0 | 0 | 0 |
| | 10-3 | 9 | 8 | 11 | 0 | 0 | 0 |
| | 10-4 | 0 | 0 | 2 | 0 | 0 | 0 |

A = Microbial inocculation (log). Reisolation per microbial carrier in dilution stage.
B = Microbial inocculation

TABLE XLV

| PSEUDOMONAS AERUGINOSA MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.83 | 5.72 | 5.70 | 5.83 | 5.72 | 5.70 |
| | B | 4.19 | 4.17 | 4.12 | U | U | U |
| | C | 1.64 | 1.55 | 1.58 | >3.83 | >3.72 | >3.70 |
| 7 | A | 5.65 | 5.64 | 5.59 | 5.65 | 5.64 | 5.59 |
| | B | 3.88 | 3.86 | 3.83 | U | U | U |
| | C | 1.77 | 1.78 | 1.76 | >3.65 | >3.64 | >3.59 |
| 14 | A | 5.55 | 5.54 | 5.51 | 5.55 | 5.54 | 5.51 |
| | B | 3.83 | 3.83 | 3.78 | U | U | U |
| | C | 1.72 | 1.71 | 1.73 | >3.55 | >3.54 | >3.51 |
| 21 | A | 5.57 | 5.55 | 5.50 | 5.57 | 5.55 | 5.50 |
| | B | 3.76 | 3.66 | 3.63 | U | U | U |
| | C | 1.81 | 1.89 | 1.87 | >3.57 | >3.55 | >3.50 |

A = Microbial inocculation
B = Reisolation
C = Reduction
U = No microbes reisolatable. Value below limit of detection of two-log stages.

TABLE XLVI

| PSEUDOMONAS AERUGINOSA MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.83 | 5.72 | 5.70 | 5.83 | 5.72 | 5.70 |
| | 10-2 | 157 | 149 | 132 | 0 | 0 | 0 |
| | 10-3 | 21 | 16 | 17 | 0 | 0 | 0 |
| | 10-4 | 3 | 2 | 1 | 0 | 0 | 0 |
| 7 | B | 5.65 | 5.64 | 5.59 | 5.65 | 5.64 | 5.59 |
| | 10-2 | 76 | 73 | 69 | 0 | 0 | 0 |
| | 10-3 | 9 | 8 | 4 | 0 | 0 | 0 |
| | 10-4 | 0 | 0 | 0 | 0 | 0 | 0 |
| 14 | B | 5.55 | 5.54 | 5.51 | 5.55 | 5.54 | 5.51 |
| | 10-2 | 68 | 69 | 61 | 0 | 0 | 0 |
| | 10-3 | 8 | 5 | 6 | 0 | 0 | 0 |
| | 10-4 | 0 | 0 | 0 | 0 | 0 | 0 |
| 21 | B | 5.57 | 5.55 | 5.50 | 5.57 | 5.55 | 5.50 |
| | 10-2 | 58 | 46 | 43 | 0 | 0 | 0 |
| | 10-3 | 7 | 6 | 3 | 0 | 0 | 0 |
| | 10-4 | 0 | 0 | 0 | 0 | 0 | 0 |
| A = Microbial inocculation (log). Reisolation per microbial carrier in dilution stage. B = Microbial inocculation | | | | | | | |

TABLE XLVII

| PSEUDOMONAS AERUGINOSA MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.83 | 5.72 | 5.70 | 5.83 | 5.72 | 5.70 |
| | B | 4.24 | 4.21 | 4.15 | U | U | U |
| | C | 1.59 | 1.51 | 1.55 | >3.83 | >3.72 | >3.70 |
| 7 | A | 5.65 | 5.64 | 5.59 | 5.65 | 5.64 | 5.59 |
| | B | 3.97 | 3.96 | 3.94 | U | U | U |
| | C | 1.68 | 1.68 | 1.65 | >3.65 | >3.64 | >3.59 |
| 14 | A | 5.55 | 5.54 | 5.51 | 5.55 | 5.54 | 5.51 |
| | B | 3.78 | 3.79 | 3.76 | U | U | U |
| | C | 1.77 | 1.75 | 1.75 | >3.55 | >3.54 | >3.51 |
| 21 | A | 5.57 | 5.55 | 5.50 | 5.57 | 5.55 | 5.50 |
| | B | 3.93 | 3.89 | 3.89 | U | U | U |
| | C | 1.64 | 1.66 | 1.61 | >3.57 | >3.55 | >3.50 |
| A = Microbial inocculation<br>B = Reisolation<br>C = Reduction<br>U = No microbes reisolatable. Value below limit of detection of two-log stages. | | | | | | | |

TABLE XLVIII

| PSEUDOMONAS AERUGINOSA MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.83 | 5.72 | 5.70 | 5.83 | 5.72 | 5.70 |
| | 10-2 | 174 | 163 | 142 | 0 | 0 | 0 |
| | 10-3 | 25 | 18 | 16 | 0 | 0 | 0 |
| | 10-4 | 6 | 2 | 2 | 0 | 0 | 0 |
| 7 | B | 5.65 | 5.64 | 5.59 | 5.65 | 5.64 | 5.59 |
| | 10-2 | 94 | 92 | 88 | 0 | 0 | 0 |
| | 10-3 | 11 | 12 | 9 | 0 | 0 | 0 |
| | 10-4 | 2 | 2 | 1 | 0 | 0 | 0 |
| 14 | B | 5.55 | 5.54 | 5.51 | 5.55 | 5.54 | 5.51 |
| | 10-2 | 61 | 62 | 58 | 0 | 0 | 0 |
| | 10-3 | 8 | 7 | 9 | 0 | 0 | 0 |
| | 10-4 | 0 | 0 | 0 | 0 | 0 | 0 |
| 21 | B | 5.57 | 5.55 | 5.50 | 5.57 | 5.55 | 5.50 |
| | 10-2 | 87 | 78 | 78 | 0 | 0 | 0 |
| | 10-3 | 11 | 9 | 8 | 0 | 0 | 0 |
| | 10-4 | 2 | 0 | 0 | 0 | 0 | 0 |
| A = Microbial inocculation (log). Reisolation per microbial carrier in dilution stage.<br>B = Microbial inocculation | | | | | | | |

TABLE XLIX

| KLEBSIELLA PNEUMONIAE MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.66 | 5.64 | 5.56 | 5.66 | 5.64 | 5.56 |
| | B | 3.79 | 3.72 | 3.66 | 2.30 | 2.30 | 2.30 |
| | C | 1.87 | 1.92 | 1.90 | >3.36 | >3.34 | >3.26 |
| 7 | A | 5.57 | 5.57 | 5.55 | 5.57 | 5.57 | 5.55 |
| | B | 3.69 | 3.62 | 3.51 | U | U | U |
| | C | 1.88 | 1.95 | 2.04 | >3.57 | >3.57 | >3.55 |
| 14 | A | 5.50 | 5.50 | 5.49 | 5.50 | 5.50 | 5.49 |
| | B | 3.74 | 3.71 | 3.64 | U | U | U |
| | C | 1.86 | 1.79 | 1.85 | >3.50 | >3.50 | >3.49 |
| 21 | A | 5.51 | 5.46 | 5.38 | 5.51 | 5.46 | 5.38 |
| | B | 3.76 | 3.79 | 3.63 | U | U | U |
| | C | 1.75 | 1.67 | 1.75 | >3.51 | >3.46 | >3.38 |

A = Microbial inocculation
B = Reisolation
C = Reduction
U = No microbes reisolatable. Value below limit of detection of two-log stages.

TABLE L

| KLEBSIELLA PNEUMONIAE MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.66 | 5.64 | 5.56 | 5.66 | 5.64 | 5.56 |
| | 10-2 | 62 | 53 | 46 | 2 | 2 | 2 |
| | 10-3 | 7 | 8 | 6 | 0 | 0 | 0 |
| | 10-4 | 0 | 0 | 0 | 0 | 0 | 0 |
| 7 | B | 5.57 | 5.57 | 5.55 | 5.57 | 5.57 | 5.55 |
| | 10-2 | 49 | 42 | 33 | 0 | 0 | 0 |
| | 10-3 | 6 | 4 | 4 | 0 | 0 | 0 |
| | 10-4 | 0 | 0 | 0 | 0 | 0 | 0 |
| 14 | B | 5.50 | 5.50 | 5.49 | 5.50 | 5.50 | 5.49 |
| | 10-2 | 56 | 52 | 44 | 0 | 0 | 0 |
| | 10-3 | 8 | 7 | 5 | 0 | 0 | 0 |
| | 10-4 | 0 | 0 | 0 | 0 | 0 | 0 |
| 21 | B | 5.51 | 5.46 | 5.38 | 5.51 | 5.46 | 5.38 |
| | 10-2 | 58 | 62 | 43 | 0 | 0 | 0 |
| | 10-3 | 8 | 11 | 7 | 0 | 0 | 0 |
| | 10-4 | 1 | 2 | 1 | 0 | 0 | 0 |
| A = Microbial inocculation (log). Reisolation per microbial carrier in dilution stage. B = Microbial inocculation | | | | | | | |

TABLE LI

| KLEBSIELLA PNEUNOMIAE MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.66 | 5.64 | 5.56 | 5.66 | 5.64 | 5.56 |
| | B | 3.81 | 3.67 | 3.61 | U | U | U |
| | C | 1.85 | 1.97 | 1.95 | >3.66 | >3.64 | >3.56 |
| 7 | A | 5.57 | 5.57 | 5.55 | 5.57 | 5.57 | 5.55 |
| | B | 3.73 | 3.71 | 3.69 | U | U | U |
| | C | 1.84 | 1.86 | 1.86 | >3.57 | >3.57 | >3.55 |
| 14 | A | 5.50 | 5.50 | 5.49 | 5.50 | 5.50 | 5.49 |
| | B | 3.74 | 3.76 | 3.67 | U | U | U |
| | C | 1.76 | 1.74 | 1.82 | >3.50 | >3.50 | >3.49 |
| 21 | A | 5.51 | 5.46 | 5.38 | 5.51 | 5.46 | 5.38 |
| | B | 3.80 | 3.79 | 3.76 | U | U | U |
| | C | 1.71 | 1.67 | 1.62 | >3.51 | >3.46 | >3.38 |

A = Microbial inocculation
B = Reisolation
C = Reduction
U = No microbes reisolatable. Value below limit of detection of two-log stages.

TABLE LII

| KLEBSIELLA PNEUMONIAE MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.66 | 5.64 | 5.56 | 5.66 | 5.64 | 5.56 |
| | 10-2 | 66 | 47 | 41 | 0 | 0 | 0 |
| | 10-3 | 8 | 5 | 5 | 0 | 0 | 0 |
| | 10-4 | 0 | 0 | 0 | 0 | 0 | 0 |
| 7 | B | 5.57 | 5.57 | 5.55 | 5.57 | 5.57 | 5.55 |
| | 10-2 | 54 | 52 | 49 | 0 | 0 | 0 |
| | 10-3 | 9 | 5 | 3 | 0 | 0 | 0 |
| | 10-4 | 1 | 0 | 0 | 0 | 0 | 0 |
| 14 | B | 5.50 | 5.50 | 5.49 | 5.50 | 5.50 | 5.49 |
| | 10-2 | 56 | 58 | 47 | 0 | 0 | 0 |
| | 10-3 | 7 | 9 | 6 | 0 | 0 | 0 |
| | 10-4 | 0 | 0 | 0 | 0 | 0 | 0 |
| 21 | B | 5.51 | 5.46 | 5.38 | 5.51 | 5.46 | 5.38 |
| | 10-2 | 64 | 62 | 58 | 0 | 0 | 0 |
| | 10-3 | 8 | 7 | 4 | 0 | 0 | 0 |
| | 10-4 | 0 | 0 | 0 | 0 | 0 | 0 |
| A = Microbial inocculation (log). Reisolation per microbial carrier in dilution stage.<br>B = Microbial inocculation | | | | | | | |

TABLE LIII

| KLEBSIELLA PNEUMONIAE MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.66 | 5.64 | 5.56 | 5.66 | 5.64 | 5.56 |
| | B | 3.72 | 3.66 | 3.53 | U | U | U |
| | C | 1.94 | 1.98 | 2.03 | >3.66 | >3.64 | >3.56 |
| 7 | A | 5.57 | 5.57 | 5.55 | 5.57 | 5.57 | 5.55 |
| | B | 3.71 | 3.66 | 3.59 | U | U | U |
| | C | 1.84 | 1.91 | 1.96 | >3.57 | >3.57 | >3.55 |
| 14 | A | 5.50 | 5.50 | 5.49 | 5.50 | 5.50 | 5.49 |
| | B | 3.69 | 3.67 | 3.57 | U | U | U |
| | C | 1.81 | 1.83 | 1.92 | >3.50 | >3.50 | >3.49 |
| 21 | A | 5.51 | 5.46 | 5.38 | 5.51 | 5.46 | 5.38 |
| | B | 3.83 | 3.80 | 3.75 | U | U | U |
| | C | 1.68 | 1.66 | 1.63 | >3.51 | >3.46 | >3.38 |
| A = Microbial inocculation<br>B = Reisolation<br>C = Reduction<br>U = No microbes reisolatable. Value below limit of detection of two-log stages. | | | | | | | |

TABLE LIV

| KLEBSIELLA PNEUMONIAE MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.66 | 5.64 | 5.56 | 5.66 | 5.64 | 5.56 |
| | 10-2 | 53 | 46 | 34 | 0 | 0 | 0 |
| | 10-3 | 7 | 6 | 3 | 0 | 0 | 0 |
| | 10-4 | 0 | 0 | 0 | 0 | 0 | 0 |
| 7 | B | 5.57 | 5.57 | 5.55 | 5.57 | 5.57 | 5.55 |
| | 10-2 | 52 | 46 | 39 | 0 | 0 | 0 |
| | 10-3 | 6 | 6 | 4 | 0 | 0 | 0 |
| | 10-4 | 0 | 0 | 0 | 0 | 0 | 0 |
| 14 | B | 5.50 | 5.50 | 5.49 | 5.50 | 5.50 | 5.49 |
| | 10-2 | 49 | 47 | 38 | 0 | 0 | 0 |
| | 10-3 | 6 | 7 | 5 | 0 | 0 | 0 |
| | 10-4 | 0 | 0 | 0 | 0 | 0 | 0 |
| 21 | B | 5.51 | 5.46 | 5.38 | 5.51 | 5.46 | 5.38 |
| | 10-2 | 68 | 64 | 57 | 0 | 0 | 0 |
| | 10-3 | 9 | 8 | 5 | 0 | 0 | 0 |
| | 10-4 | 0 | 0 | 0 | 0 | 0 | 0 |
| A = Microbial inocculation (log). Reisolation per microbial carrier in dilution stage.<br>B = Microbial inocculation | | | | | | | |

TABLE LV

| KLEBSIELLA PNEUNOMIAE MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.66 | 5.64 | 5.56 | 5.66 | 5.64 | 5.56 |
| | B | 3.68 | 3.64 | 3.59 | U | U | U |
| | C | 1.98 | 2.00 | 1.97 | >3.66 | >3.64 | >3.56 |
| 7 | A | 5.57 | 5.57 | 5.55 | 5.57 | 5.57 | 5.55 |
| | B | 3.76 | 3.73 | 3.67 | U | U | U |
| | C | 1.81 | 1.84 | 1.88 | >3.57 | >3.57 | >3.55 |
| 14 | A | 5.50 | 5.50 | 5.49 | 5.50 | 5.50 | 5.49 |
| | B | 3.67 | 3.66 | 3.62 | U | U | U |
| | C | 1.83 | 1.84 | 1.87 | >3.50 | >3.50 | >3.49 |
| 21 | A | 5.51 | 5.46 | 5.38 | 5.51 | 5.46 | 5.38 |
| | B | 3.76 | 3.73 | 3.72 | U | U | U |
| | C | 1.75 | 1.73 | 1.66 | >3.51 | >3.46 | >3.38 |
| A = Microbial inocculation<br>B = Reisolation<br>C = Reduction<br>U = No microbes reisolatable. Value below limit of detection of two-log stages. | | | | | | | |

TABLE LVI

| KLEBSIELLA PNEUMONIAE MICROBIAL GROWTH REDUCTION OF TREATED AND UNTREATED NYLON HEAVY-DUTY FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.66 | 5.64 | 5.56 | 5.66 | 5.64 | 5.56 |
| | 10-2 | 48 | 44 | 39 | 0 | 0 | 0 |
| | 10-3 | 7 | 5 | 4 | 0 | 0 | 0 |
| | 10-4 | 0 | 0 | 0 | 0 | 0 | 0 |
| 7 | B | 5.57 | 5.57 | 5.55 | 5.57 | 5.57 | 5.55 |
| | 10-2 | 58 | 54 | 47 | 0 | 0 | 0 |
| | 10-3 | 7 | 5 | 5 | 0 | 0 | 0 |
| | 10-4 | 0 | 0 | 0 | 0 | 0 | 0 |
| 14 | B | 5.50 | 5.50 | 5.49 | 5.50 | 5.50 | 5.49 |
| | 10-2 | 47 | 46 | 42 | 0 | 0 | 0 |
| | 10-3 | 5 | 6 | 4 | 0 | 0 | 0 |
| | 10-4 | 0 | 0 | 0 | 0 | 0 | 0 |
| 21 | B | 5.51 | 5.46 | 5.38 | 5.51 | 5.46 | 5.38 |
| | 10-2 | 58 | 54 | 53 | 0 | 0 | 0 |
| | 10-3 | 6 | 7 | 6 | 0 | 0 | 0 |
| | 10-4 | 0 | 0 | 0 | 0 | 0 | 0 |
| A = Microbial inocculation (log). Reisolation per microbial carrier in dilution stage.<br>B = Microbial inocculation | | | | | | | |

TABLE LVII

| SALMONELLA TYPHI MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.20 | 5.25 | 5.20 | 5.20 | 5.25 | 5.20 |
| | B | 3.32 | 3.23 | 2.95 | U | U | U |
| | C | 1.88 | 2.02 | 2.25 | >3.20 | >3.25 | >3.20 |
| 7 | A | 5.14 | 5.17 | 5.11 | 5.14 | 5.17 | 5.11 |
| | B | 3.07 | 2.90 | 2.84 | U | U | U |
| | C | 2.07 | 2.27 | 2.27 | >3.14 | >3.17 | >3.11 |
| 14 | A | 5.25 | 5.17 | 5.23 | 5.25 | 5.17 | 5.23 |
| | B | 3.51 | 3.44 | 3.43 | U | U | U |
| | C | 1.74 | 1.73 | 1.80 | >3.25 | >3.17 | >3.23 |
| 21 | A | 5.20 | 5.17 | 5.07 | 5.20 | 5.17 | 5.07 |
| | B | 3.46 | 3.43 | 3.36 | U | U | U |
| | C | 1.74 | 1.74 | 1.71 | >3.20 | >3.17 | >3.07 |

A = Microbial inocculation
B = Reisolation
C = Reduction
U = No microbes reisolatable. Value below limit of detection of two-log stages.

TABLE LVIII

| SALMONELLA TYPHI MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.20 | 5.25 | 5.20 | 5.20 | 5.25 | 5.20 |
| | 10-2 | 21 | 17 | 9 | 0 | 0 | 0 |
| | 10-3 | 3 | 2 | 1 | 0 | 0 | 0 |
| | 10-4 | 0 | 0 | 0 | 0 | 0 | 0 |
| 7 | B | 5.14 | 5.17 | 5.11 | 5.14 | 5.17 | 5.11 |
| | 10-2 | 12 | 8 | 7 | 0 | 0 | 0 |
| | 10-3 | 1 | 1 | 1 | 0 | 0 | 0 |
| | 10-4 | 0 | 0 | 0 | 0 | 0 | 0 |
| 14 | B | 5.25 | 5.17 | 5.23 | 5.25 | 5.17 | 5.23 |
| | 10-2 | 33 | 28 | 27 | 0 | 0 | 0 |
| | 10-3 | 4 | 4 | 2 | 0 | 0 | 0 |
| | 10-4 | 0 | 0 | 0 | 0 | 0 | 0 |
| 21 | B | 5.20 | 5.17 | 5.07 | 5.20 | 5.17 | 5.07 |
| | 10-2 | 29 | 27 | 23 | 0 | 0 | 0 |
| | 10-3 | 4 | 3 | 3 | 0 | 0 | 0 |
| | 10-4 | 0 | 0 | 0 | 0 | 0 | 0 |
| A = Microbial inocculation (log). Reisolation per microbial carrier in dilution stage. B = Microbial inocculation | | | | | | | |

TABLE LIX

| SALMONELLA TYPHI MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.20 | 5.25 | 5.20 | 5.20 | 5.25 | 5.20 |
| | B | 3.27 | 3.34 | 3.20 | U | U | U |
| | C | 1.93 | 1.91 | 2.00 | >3.20 | >3.25 | >3.20 |
| 7 | A | 5.14 | 5.17 | 5.11 | 5.14 | 5.17 | 5.11 |
| | B | 3.14 | 3.07 | 2.90 | U | U | U |
| | C | 2.00 | 2.10 | 2.21 | >3.14 | >3.17 | >3.11 |
| 14 | A | 5.25 | 5.17 | 5.23 | 5.25 | 5.17 | 5.23 |
| | B | 3.20 | 3.07 | 2.95 | U | U | U |
| | C | 2.05 | 2.10 | 2.28 | >3.25 | >3.17 | >3.23 |
| 21 | A | 5.20 | 5.17 | 5.07 | 5.20 | 5.17 | 5.07 |
| | B | 3.32 | 3.14 | 3.14 | U | U | U |
| | C | 1.88 | 2.03 | 1.93 | >3.20 | >3.17 | >3.07 |

A = Microbial inocculation

B = Reisolation

C = Reduction

U = No microbes reisolatable. Value below limit of detection of two-log stages.

TABLE LX

| SALMONELLA TYPHI MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.20 | 5.25 | 5.20 | 5.20 | 5.25 | 5.20 |
| | 10-2 | 19 | 22 | 16 | 0 | 0 | 0 |
| | 10-3 | 2 | 3 | 2 | 0 | 0 | 0 |
| | 10-4 | 0 | 0 | 0 | 0 | 0 | 0 |
| 7 | B | 5.14 | 5.17 | 5.11 | 5.14 | 5.17 | 5.11 |
| | 10-2 | 14 | 12 | 8 | 0 | 0 | 0 |
| | 10-3 | 3 | 1 | 0 | 0 | 0 | 0 |
| | 10-4 | 0 | 0 | 0 | 0 | 0 | 0 |
| 14 | B | 5.25 | 5.17 | 5.23 | 5.25 | 5.17 | 5.23 |
| | 10-2 | 16 | 12 | 9 | 0 | 0 | 0 |
| | 10-3 | 2 | 1 | 0 | 0 | 0 | 0 |
| | 10-4 | 0 | 0 | 0 | 0 | 0 | 0 |
| 21 | B | 5.20 | 5.17 | 5.07 | 5.20 | 5.17 | 5.07 |
| | 10-2 | 21 | 14 | 14 | 0 | 0 | 0 |
| | 10-3 | 2 | 2 | 1 | 0 | 0 | 0 |
| | 10-4 | 0 | 0 | 0 | 0 | 0 | 0 |
| A = Microbial inocculation (log). Reisolation per microbial carrier in dilution stage. B = Microbial inocculation | | | | | | | |

TABLE LXI

| SALMONELLA TYPHI MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.20 | 5.25 | 5.20 | 5.20 | 5.25 | 5.20 |
| | B | 3.27 | 3.32 | 3.20 | U | U | U |
| | C | 1.93 | 1.93 | 2.00 | >3.20 | >3.25 | >3.20 |
| 7 | A | 5.14 | 5.17 | 5.11 | 5.14 | 5.17 | 5.11 |
| | B | 3.11 | 3.04 | 3.00 | U | U | U |
| | C | 2.03 | 2.13 | 2.11 | >3.14 | >3.17 | >3.11 |
| 14 | A | 5.25 | 5.17 | 5.23 | 5.25 | 5.17 | 5.23 |
| | B | 3.14 | 3.04 | 2.84 | U | U | U |
| | C | 2.11 | 2.13 | 2.39 | >3.25 | >3.17 | >3.23 |
| 21 | A | 5.20 | 5.17 | 5.07 | 5.20 | 5.17 | 5.07 |
| | B | 3.32 | 3.20 | 3.14 | U | U | U |
| | C | 1.88 | 1.97 | 1.93 | >3.20 | >3.17 | >3.07 |

A = Microbial inocculation

B = Reisolation

C = Reduction

U = No microbes reisolatable. Value below limit of detection of two-log stages.

TABLE LXII

| SALMONELLA TYPHI MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.20 | 5.25 | 5.20 | 5.20 | 5.25 | 5.20 |
| | 10-2 | 19 | 21 | 16 | 0 | 0 | 0 |
| | 10-3 | 2 | 3 | 2 | 0 | 0 | 0 |
| | 10-4 | 0 | 0 | 0 | 0 | 0 | 0 |
| 7 | B | 5.14 | 5.17 | 5.11 | 5.14 | 5.17 | 5.11 |
| | 10-2 | 13 | 11 | 10 | 0 | 0 | 0 |
| | 10-3 | 1 | 0 | 0 | 0 | 0 | 0 |
| | 10-4 | 0 | 0 | 0 | 0 | 0 | 0 |
| 14 | B | 5.25 | 5.17 | 5.23 | 5.25 | 5.17 | 5.23 |
| | 10-2 | 14 | 11 | 7 | 0 | 0 | 0 |
| | 10-3 | 2 | 1 | 1 | 0 | 0 | 0 |
| | 10-4 | 0 | 0 | 0 | 0 | 0 | 0 |
| 21 | B | 5.20 | 5.17 | 5.07 | 5.20 | 5.17 | 5.07 |
| | 10-2 | 21 | 16 | 14 | 0 | 0 | 0 |
| | 10-3 | 2 | 1 | 2 | 0 | 0 | 0 |
| | 10-4 | 0 | 0 | 0 | 0 | 0 | 0 |
| A = Microbial inocculation (log). Reisolation per microbial carrier in dilution stage.<br>B = Microbial inocculation | | | | | | | |

TABLE LXIII

| SALMONELLA TYPHI MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.20 | 5.25 | 5.20 | 5.20 | 5.25 | 5.20 |
| | B | 3.25 | 3.23 | 3.04 | U | U | U |
| | C | 1.95 | 2.02 | 2.16 | >3.20 | >3.25 | >3.20 |
| 7 | A | 5.14 | 5.17 | 5.11 | 5.14 | 5.17 | 5.11 |
| | B | 3.20 | 3.04 | 3.14 | U | U | U |
| | C | 1.94 | 2.13 | 1.97 | >3.14 | >3.17 | >3.11 |
| 14 | A | 5.25 | 5.17 | 5.23 | 5.25 | 5.17 | 5.23 |
| | B | 3.49 | 3.44 | 3.38 | U | U | U |
| | C | 1.76 | 1.73 | 1.85 | >3.25 | >3.17 | >3.23 |
| 21 | A | 5.20 | 5.17 | 5.07 | 5.20 | 5.17 | 5.07 |
| | B | 3.38 | 3.36 | 3.30 | U | U | U |
| | C | 1.82 | 1.81 | 1.77 | >3.20 | >3.17 | >3.07 |
| A = Microbial inocculation<br>B = Reisolation<br>C = Reduction<br>U = No microbes reisolatable. Value below limit of detection of two-log stages. | | | | | | | |

TABLE LXIV

| SALMONELLA TYPHI MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.20 | 5.25 | 5.20 | 5.20 | 5.25 | 5.20 |
| | 10-2 | 18 | 17 | 11 | 0 | 0 | 0 |
| | 10-3 | 2 | 2 | 2 | 0 | 0 | 0 |
| | 10-4 | 0 | 0 | 0 | 0 | 0 | 0 |
| 7 | B | 5.14 | 5.17 | 5.11 | 5.14 | 5.17 | 5.11 |
| | 10-2 | 16 | 11 | 14 | 0 | 0 | 0 |
| | 10-3 | 2 | 1 | 0 | 0 | 0 | 0 |
| | 10-4 | 0 | 0 | 0 | 0 | 0 | 0 |
| 14 | B | 5.25 | 5.17 | 5.23 | 5.25 | 5.17 | 5.23 |
| | 10-2 | 31 | 28 | 24 | 0 | 0 | 0 |
| | 10-3 | 4 | 4 | 3 | 0 | 0 | 0 |
| | 10-4 | 0 | 0 | 0 | 0 | 0 | 0 |
| 21 | B | 5.20 | 5.17 | 5.07 | 5.20 | 5.17 | 5.07 |
| | 10-2 | 24 | 23 | 20 | 0 | 0 | 0 |
| | 10-3 | 3 | 3 | 2 | 0 | 0 | 0 |
| | 10-4 | 0 | 0 | 0 | 0 | 0 | 0 |
| A = Microbial inocculation (log). Reisolation per microbial carrier in dilution stage. B = Microbial inocculation | | | | | | | |

TABLE LXV

| STREPTOCOCCUS MUTANS MICROBIAL GROWTH REDUCTION OF TREATED AND UNTREATED HIGH-PILE CUT NYLON FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.91 | 5.84 | 5.73 | 5.91 | 5.84 | 5.20 |
| | B | 3.50 | 3.39 | 3.20 | U | U | U |
| | C | 2.41 | 2.45 | 2.53 | >3.91 | >3.84 | >3.73 |
| 7 | A | 5.64 | 5.62 | 5.57 | 5.64 | 5.62 | 5.57 |
| | B | 3.32 | 3.25 | 3.20 | U | U | U |
| | C | 2.32 | 2.37 | 2.37 | >3.64 | >3.62 | >3.57 |
| 14 | A | 5.55 | 5.52 | 5.49 | 5.55 | 5.52 | 5.49 |
| | B | 3.32 | 3.27 | 3.27 | U | U | U |
| | C | 2.23 | 2.25 | 2.22 | >3.55 | >3.52 | >3.49 |
| 21 | A | 5.50 | 5.52 | 5.49 | 5.50 | 5.52 | 5.49 |
| | B | 3.44 | 3.38 | 3.32 | U | U | U |
| | C | 2.06 | 2.14 | 2.17 | >3.50 | >3.52 | >3.49 |

A = Microbial inocculation
B = Reisolation
C = Reduction
U = No microbes reisolatable. Value below limit of detection of two-log stages.

TABLE LXVI

| STREPTOCOCCUS MUTANS MICROBIAL GROWTH REDUCTION OF TREATED AND UNTREATED HIGH-PILE CUT NYLON TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.91 | 5.84 | 5.73 | 5.91 | 5.84 | 5.73 |
| | 10-2 | 32 | 25 | 16 | 0 | 0 | 0 |
| | 10-3 | 4 | 3 | 2 | 0 | 0 | 0 |
| | 10-4 | 0 | 0 | 0 | 0 | 0 | 0 |
| 7 | B | 5.64 | 5.62 | 5.57 | 5.64 | 5.62 | 5.57 |
| | 10-2 | 21 | 18 | 16 | 0 | 0 | 0 |
| | 10-3 | 3 | 2 | 1 | 0 | 0 | 0 |
| | 10-4 | 0 | 0 | 0 | 0 | 0 | 0 |
| 14 | B | 5.55 | 5.52 | 5.49 | 5.55 | 5.52 | 5.49 |
| | 10-2 | 21 | 19 | 19 | 0 | 0 | 0 |
| | 10-3 | 3 | 3 | 2 | 0 | 0 | 0 |
| | 10-4 | 0 | 0 | 0 | 0 | 0 | 0 |
| 21 | B | 5.50 | 5.52 | 5.49 | 5.50 | 5.52 | 5.49 |
| | 10-2 | 28 | 24 | 21 | 0 | 0 | 0 |
| | 10-3 | 3 | 3 | 2 | 0 | 0 | 0 |
| | 10-4 | 0 | 0 | 0 | 0 | 0 | 0 |
| A = Microbial inocculation (log). Reisolation per microbial carrier in dilution stage. B = Microbial inocculation | | | | | | | |

TABLE LXVII

| STREPTOCOCCUS MUTANS MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.91 | 5.84 | 5.73 | 5.91 | 5.84 | 5.73 |
| | B | 3.62 | 3.51 | 3.41 | U | U | U |
| | C | 2.29 | 2.33 | 2.32 | >3.91 | >3.84 | >3.73 |
| 7 | A | 5.64 | 5.62 | 5.57 | 5.64 | 5.62 | 5.57 |
| | B | 3.41 | 3.25 | 3.14 | U | U | U |
| | C | 2.23 | 2.37 | 2.43 | >3.64 | >3.62 | >3.57 |
| 14 | A | 5.55 | 5.52 | 5.49 | 5.55 | 5.52 | 5.49 |
| | B | 3.27 | 3.25 | 3.23 | U | U | U |
| | C | 2.28 | 2.27 | 2.26 | >3.55 | >3.52 | >3.49 |
| 21 | A | 5.50 | 5.52 | 5.49 | 5.50 | 5.52 | 5.49 |
| | B | 3.36 | 3.23 | 3.07 | U | U | U |
| | C | 2.14 | 2.29 | 2.42 | >3.50 | >3.52 | >3.49 |

A = Microbial inocculation

B = Reisolation

C = Reduction

U = No microbes reisolatable. Value below limit of detection of two-log stages.

TABLE LXVIII

| STREPTOCOCCUS MUTANS MICROBIAL GROWTH REDUCTION OF TREATED AND UNTREATED NYLON FINE VELOUR TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.91 | 5.84 | 5.73 | 5.91 | 5.84 | 5.73 |
| | 10-2 | 42 | 33 | 26 | 0 | 0 | 0 |
| | 10-3 | 4 | 4 | 3 | 0 | 0 | 0 |
| | 10-4 | 0 | 0 | 0 | 0 | 0 | 0 |
| 7 | B | 5.64 | 5.62 | 5.57 | 5.64 | 5.62 | 5.57 |
| | 10-2 | 16 | 18 | 14 | 0 | 0 | 0 |
| | 10-3 | 4 | 3 | 1 | 0 | 0 | 0 |
| | 10-4 | 0 | 0 | 0 | 0 | 0 | 0 |
| 14 | B | 5.55 | 5.52 | 5.49 | 5.55 | 5.52 | 5.49 |
| | 10-2 | 19 | 18 | 17 | 0 | 0 | 0 |
| | 10-3 | 3 | 3 | 1 | 0 | 0 | 0 |
| | 10-4 | 0 | 0 | 0 | 0 | 0 | 0 |
| 21 | B | 5.50 | 5.52 | 5.49 | 5.50 | 5.52 | 5.49 |
| | 10-2 | 23 | 17 | 12 | 0 | 0 | 0 |
| | 10-3 | 3 | 2 | 1 | 0 | 0 | 0 |
| | 10-4 | 0 | 0 | 0 | 0 | 0 | 0 |
| A = Microbial inocculation (log). Reisolation per microbial carrier in dilution stage.<br>B = Microbial inocculation | | | | | | | |

TABLE LXIX

| STREPTOCOCCUS MUTANS MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.91 | 5.84 | 5.73 | 5.91 | 5.84 | 5.73 |
| | B | 3.20 | 3.07 | 2.95 | U | U | U |
| | C | 2.71 | 2.77 | 2.78 | >3.91 | >3.84 | >3.73 |
| 7 | A | 5.64 | 5.62 | 5.57 | 5.64 | 5.62 | 5.57 |
| | B | 3.32 | 3.23 | 3.14 | U | U | U |
| | C | 2.32 | 2.39 | 2.43 | >3.64 | >3.62 | >3.57 |
| 14 | A | 5.55 | 5.52 | 5.49 | 5.55 | 5.52 | 5.49 |
| | B | 3.17 | 3.17 | 3.17 | U | U | U |
| | C | 2.38 | 2.35 | 2.32 | >3.55 | >3.52 | >3.49 |
| 21 | A | 5.50 | 5.52 | 5.49 | 5.50 | 5.52 | 5.49 |
| | B | 3.14 | 3.07 | 3.04 | U | U | U |
| | C | 2.36 | 2.45 | 2.45 | >3.50 | >3.52 | >3.49 |

A = Microbial inocculation
B = Reisolation
C = Reduction
U = No microbes reisolatable. Value below limit of detection of two-log stages.

TABLE LXX

| STREPTOCOCCUS MUTANS MICROBIAL GROWTH REDUCTION OF TREATED AND UNTREATED NYLON LIGHT LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.91 | 5.84 | 5.73 | 5.91 | 5.84 | 5.73 |
| | 10-2 | 16 | 12 | 9 | 0 | 0 | 0 |
| | 10-3 | 2 | 1 | 1 | 0 | 0 | 0 |
| | 10-4 | 0 | 0 | 0 | 0 | 0 | 0 |
| 7 | B | 5.64 | 5.62 | 5.57 | 5.64 | 5.62 | 5.57 |
| | 10-2 | 21 | 17 | 14 | 0 | 0 | 0 |
| | 10-3 | 3 | 2 | 2 | 0 | 0 | 0 |
| | 10-4 | 0 | 0 | 0 | 0 | 0 | 0 |
| 14 | B | 5.555 | 5.52 | 5.49 | 5.55 | 5.52 | 5.49 |
| | 10-2 | 15 | 15 | 15 | 0 | 0 | 0 |
| | 10-3 | 2 | 2 | 1 | 0 | 0 | 0 |
| | 10-4 | 0 | 0 | 0 | 0 | 0 | 0 |
| 21 | B | 5.50 | 5.52 | 5.49 | 5.50 | 5.52 | 5.49 |
| | 10-2 | 14 | 12 | 11 | 0 | 0 | 0 |
| | 10-3 | 2 | 1 | 1 | 0 | 0 | 0 |
| | 10-4 | 0 | 0 | 0 | 0 | 0 | 0 |
| A = Microbial inocculation (log). Reisolation per microbial carrier in dilution stage.<br>B = Microbial inocculation | | | | | | | |

TABLE LXXI

| STREPTOCOCCUS MUTANS MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | Legend | Untreated | | | TMS Treated | | |
| | | Run 1 | Run 2 | Run 3 | Run 1 | Run 2 | Run 3 |
| 0 | A | 5.91 | 5.84 | 5.73 | 5.91 | 5.84 | 5.73 |
| | B | 3.27 | 3.11 | 3.07 | U | U | U |
| | C | 2.64 | 2.73 | 2.66 | >3.91 | >3.84 | >3.73 |
| 7 | A | 5.64 | 5.62 | 5.57 | 5.64 | 5.62 | 5.57 |
| | B | 3.38 | 3.07 | 2.90 | U | U | U |
| | C | 2.26 | 2.55 | 2.67 | >3.64 | >3.62 | >3.57 |
| 14 | A | 5.55 | 5.52 | 5.49 | 5.55 | 5.52 | 5.49 |
| | B | 3.27 | 3.23 | 3.07 | U | U. | U |
| | C | 2.28 | 2.29 | 2.42 | >3.55 | >3.52 | >3.49 |
| 21 | A | 5.50 | 5.52 | 5.49 | 5.50 | 5.52 | 5.49 |
| | B | 3.27 | 3.25 | 3.25 | U | U | U |
| | C | 2.23 | 2.27 | 2.24 | >3.50 | >3.52 | >3.49 |

A = Microbial inocculation
B = Reisolation
C = Reduction
U = No microbes reisolatable. Value below limit of detection of two-log stages.

TABLE LXXII

| STREPTOCOCCUS MUTANS MICROBIAL GROWTH REDUCTION OF TREATED AND UNTREATED NYLON HEAVY-DUTY LOOP FABRIC TEST SURFACES | | | | | | | |
|---|---|---|---|---|---|---|---|
| Shampoo Treatments | A | Untreated | | | TMS Treated | | |
| | | Run 1 | Run 2 | Run 3 | Run 1 | Run 2 | Run 3 |
| 0 | B | 5.91 | 5.84 | 5.73 | 5.91 | 5.84 | 5.73 |
| | 10-2 | 19 | 13 | 11 | 0 | 0 | 0 |
| | 10-3 | 2 | 2 | 1 | 0 | 0 | 0 |
| | 10-4 | 0 | 0 | 0 | 0 | 0 | 0 |
| 7 | B | 5.64 | 5.62 | 5.57 | 5.64 | 5.62 | 5.57 |
| | 10-2 | 24 | 12 | 8 | 0 | 0 | 0 |
| | 10-3 | 3 | 1 | 0 | 0 | 0 | 0 |
| | 10-4 | 0 | 0 | 0 | 0 | 0 | 0 |
| 14 | B | 5.55 | 5.52 | 5.49 | 5.55 | 5.52 | 5.49 |
| | 10-2 | 19 | 17 | 12 | 0 | 0 | 0 |
| | 10-3 | 3 | 2 | 2 | 0 | 0 | 0 |
| | 10-4 | 0 | 0 | 0 | 0 | 0 | 0 |
| 21 | B | 5.50 | 5.52 | 5.49 | 5.50 | 5.52 | 5.49 |
| | 10-2 | 19 | 18 | 18 | 0 | 0 | 0 |
| | 10-3 | 3 | 2 | 2 | 0 | 0 | 0 |
| | 10-4 | 0 | 0 | 0 | 0 | 0 | 0 |
| A = Microbial inocculation (log). Reisolation per microbial carrier in dilution stage. B = Microbial inocculation | | | | | | | |

The foregoing examples, tables and tests illustrate the broad spectrum activity of the compounds of the present invention on carpeting against various microorganisms. Such compounds have been found to be effective against a number of other microorganisms, such as BACTERIA: Gram (-); Escherichia coli, Klebsiella pneumoniae, Klebsiella oxytoca, Pseudomonas aeruginosa, Pseudomonas fluorescens, Proteus mirabilis, Proteus vulgaris, Salmonella typhi, Salmonella typhimurium, Salmonella cholera suis, Enterobacter cloacae, Enterobacter aerogenes, Morganella morganii, Aeromonas hydrophila, Citrobacter freundii, Citrobacter deversus, Serratia marcescens, Serratia liquifaciens, Xanthomonas campestris, Acinetobacter calcoaceticus; Gram (+): Staphylococcus aureus, Staphylococcus epidermidis, Streptococcus mutans, Streptococcus pyogenes, Streptococcus fecalis, Micrococcus lutea, Bacillus sp. (vegetative cell); Fungi: Aspergillus niger, Aspergillus flavus, Aspergillus sydowi, Aspergillus versicolor, Aspergillus terreus, Penicillium chrysogenum, Penicillium variabile, Penicillium funiculosum, Penicillium pinophilum, Poria placenta, Aureobasidium pullulans, Gloeophyllum trabeum, Chaetomium globosum, Trichoderma viride, Trichophyton mentagrophytes; Fungi (yeasts): Candida albicans, Candida pseudotropicalis, Saccharomyces cerevisiae.

The material used herein as a filter for liquids and gases associated with the forced air system of the structure is preferably that foam composition disclosed and described in U.S. Patent No. 4,631,297, issued December 23, 1986. This patent describes a method for preparing antimicrobially effective foams using silanes as co-catalysts, co-surfactants and antimicrobial agents to give soft, resilient, fine-celled foams that are capable of reducing the number of or eliminating, microorganisms. Such foams themselves are not susceptible to attack by the microorganisms. An example is the use of $(CH_3O)_3Si(CH_2)_3N^{\oplus}(CH_3)_2C_{18}H_{37}Cl^-$ in a flexible polyurethane foam formulation to give a fine-celled, soft, resilient foam which is antimicrobially effective over a long period of time.

For purposes of the present invention, the term interior surface of the building or structure is intended to include, for example, floors and floor coverings; ceilings; ventilation ducts and conduits; rugs; carpets; walls and partitions; windows; doors; hallways; elevators; offices; conference rooms; stairways; bath and sanitary facilities; furniture, office equipment and furnishings; tables; chairs; desks; draperies; insulation; and backing materials.

The treatment of aqueous medium of the building structure that is associated with the building forced or induced air ventilation, humidification, heating and air conditioning system, such as the building cooling towers, humidifiers, air conditioners and cisterns, in order to kill and inhibit the growth of microorganisms responsible for the spread and transmission of diseases, infections and allergies, is performed by adding the organosilicon quaternary ammonium compounds of the present invention to the aqueous medium in amounts of a few parts per million concentration or a few weight percent concentration. This treatment when applied in conjunction with the filtration of the aqueous fluids and air associated with such systems, insures that many exterior as well as interior building sources of microorganisms will be prevented from contributing to the overall interior building microbial load and hence provide added protection against the spread and transmission of disease, infection and allergies. When combined with the building interior surface treatments, the spread and transmission of such maladies can be significantly reduced.

Example IV

In Alexandria, Kentucky, a thirty-thousand square foot school building was infested with mold resulting in staff and student complaints of weeping eyes, tight throats and other symptoms associated with "Sick Building Syndrome". Various solutions had been tried including the use of disinfectants over six weekly applications and changing the air handling system; however, the complaints persisted. Initial mold and bacterial counts were determined by air settling plates. The mold count was found to be 48.2 CFU and the bacterial count 12.9 CFU, expressed as colony forming units. The building was treated in accordance with the teaching of the present invention and the counts again determined. The mold count was found to be 17.9 CFU and the bacterial count 4.1 CFU, resulting in no further complaints from staff or students. This effectiveness has been monitored for over two years.

Example V

In Fort Myers, Florida, an office building was infested with mold resulting in worker absenteeism and complaints of headaches, nausea, sore throats and other symptoms of "Sick Building Syndrome". Various solutions had been tried including changing the air handling system; however, the absenteeism and the worker complaints persisted. Initial mold and bacterial counts were determined by air settling plates. The mold count was found to be 43 CFU and the bacterial count 28 CFU, expressed as colony forming units. The building was treated in accordance with the teaching of the present invention and the counts again determined. The mold count was negligible and the bacterial count found to be 8 CFU, resulting in no further complaints from workers.

Example VI

In Rochester, New York, an office building was infested with mold resulting in odor and worker complaints of burning eyes and tight chests. Various solutions had been tried including carpet cleaning and changing the air handling system; however, the complaints, odor and burning eyes and tight chests persisted. Initial mold counts were determined by air settling plates. The mold count was found to be 48 CFU expressed as colony forming units. The building was treated in accordance with the teaching of the present invention and the counts again determined. The mold count was found to be 4 CFU resulting in no further complaints even in high humidity and no further odors.

In addition to Examples IV to VI, the following fungi were isolated before treatment in a building in Charleston, South Carolina. All were controlled with treatment of the building in accordance with the teaching of the present invention. The bacterial types, analysis and identification with notations is set forth as follows:

Penicillium citrinum Thom 0.80 $a_w$, Trichoderma harzianum Refai, Aspergillus nidulans Wirt, Penicillium citrinum, Pen oxalicum, Trichoderma harzianum, Stachybotrysatracorda, Phoma herberum Westend, Cladosporium sphaero spermum Penz, Penicillium citrinum, Thom 0.80 $a_w$, Acremonium strictum gams, Sytalidium Lignicols Fesante, Streptomyces griseus, Chaetomium globosum Kunze 0.94 $a_w$, Chaetomium globosum, Phomopsis vexans sacc. 0.95 $a_w$, Cladosporium cladosporoides, Aspergillus sydowii, Chaetomium globosum, Penicillium oxalicumCurrie et Thom 0.86 $a_w$, Acremonium strictum, Aspergillus sydowii Thom et Church 0.78 $a_w$, Cladosporium cladosporoides (Fres.) deVries 0.90 $a_w$, Penicillium

restrictum, Penicillium restrictum Gilman and Abbott 0.82 $a_w$, Geotrichum candidum, Link 0.90 $a_w$, Penicillium oxalicum, Acremonium oxalicum.

In conducting treatments of building structures in accordance with the teaching of the present invention, it is contemplated to treat all surfaces of the building which are believed to harbor substantial concentrations of microorganisms. Such treatment may, for example, entail the treatment of the interior of air handling system duct-work as well as exterior surfaces. It will, of course, include the treatment of carpeting and rugs especially. Wherever practical, the building fluids including liquids and gases should be exposed to the treated foams in the form of filtration media, such as in air handling system filters and cooling tower water or humidification liquid filtration. In addition, accumulated water should be treated with the composition whether in the form of cisterns or basins and the like. Much depends on the characteristics of the particular building, but wherever possible it is contemplated to expose as much of the building structure and fluids to the compositions of the present invention as is practical under each circumstance. The treatment may be delivered from an aqueous medium or a compatible solvent system using wetting agents or without as a spray, foam, fog, wipe or wash.

It will be apparent from the foregoing that many other variations and modifications may be made in the structures, compounds, compositions and methods described herein without departing substantially from the essential features and concepts of the present invention. Accordingly, it should be clearly understood that the forms of the invention described herein are exemplary only and are not intended as limitations on the scope of the present invention.

## Claims

1. A method of inhibiting the spread, transmission and incidence, of disease, infection and allergies, characteristic of hypersensitivity pneumonitis in structures employing forced or induced air ventilation, heating, humidification and air conditioning systems, and with surfaces and furnishings conducive to and as harbors for microbial growths, in which microorganisms, their metabolic products and their somatic and reproductive cell parts, contribute to the spread, transmission and incidence, of the disease, infection and aggravate the condition of microbially mediated asthma and rhinitis allergies or initiate such allergic response, comprising reducing the level of exposure of occupants of the structure to high levels of microorganisms by treating interior surfaces and furnishings of the structure and immobilizing on the surfaces and bonding thereto a coating of an immobilized antimicrobial organosilicon quaternary ammonium compound in an amount effective to significantly destroy, reduce and inhibit the growth and multiplication of bacteria, mildew, mold, fungi, yeast, algae and other pathogenic microorganisms, which cause defacement, surface deterioriation, mildew and putrid odors; lowering the threshold level of airborne levels of microorganisms with the organosilicon quaternary ammonium compound in order to prevent recolonization and reinfestation; and utilizing the intrinsic immobilized antimicrobial activity of the treated surfaces to kill transient microbes; the immobilized antimicrobial organosilicon quaternary ammonium compound being an organosilane having the general formula selected from the group consisting of:

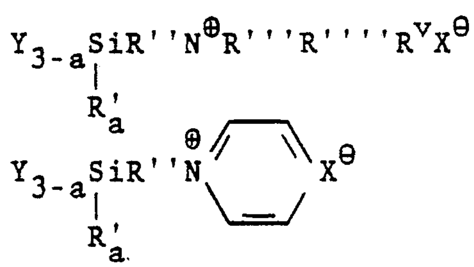

and

wherein, in each formula,

Y is R or RO where R is an alkyl radical of 1 to 4 carbon atoms or hydrogen;

a has a value of 0, 1 or 2;

$R'$ is a methyl or ethyl radical;

$R''$ is an alkylene group of 1 to 4 carbon atoms;

$R'''$, $R''''$ and $R^v$ are each independently selected from a group consisting of alkyl radicals of 1 to 18 carbon atoms, $-CH_2C_6H_5$, $-CH_2CH_2OH$, $-CH_2OH$ and $-(CH_2)_xNHC(O)R^{vi}$, wherein x has a value of from 2 to 10 and

$R^{vi}$ is a perfluoroalkyl radical having from 1 to 12 carbon atoms;
X is chloride, bromide, fluoride, iodide, acetate or tosylate.

2. A method of treating hypersensitivity pneumonitis in structures with surfaces and furnishings conducive to and as harbors for microbial growths, comprising reducing the level of exposure of occupants of the structure to high levels of microorganisms by treating interior surfaces and furnishings of the structure and immobilizing on the surfaces and bonding thereto a coating of an antimicrobial organosilicon quaternary ammonium compound in an amount effective to significantly destroy, reduce and inhibit the growth and multiplication of pathogenic microorganisms which cause defacement, surface deterioriation, mildew and putrid odors; lowering the threshold level of airborne levels of microorganisms with the organosilicon quaternary ammonium compound in order to prevent recolonization and reinfestation; and utilizing the intrinsic immobilized antimicrobial activity of the treated surfaces to kill transient microbes; the immobilized antimicrobial organosilicon quaternary ammonium compound being an organosilane having the general formula selected from the group consisting of:

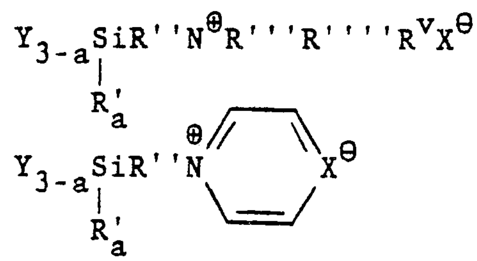

and
wherein, in each formula,
Y is R or RO where R is an alkyl radical of 1 to 4 carbon atoms or hydrogen;
a has a value of 0, 1 or 2;
$R'$ is a methyl or ethyl radical;
$R''$ is an alkylene group of 1 to 4 carbon atoms;
$R'''$, $R''''$ and $R^{v}$ are each independently selected from a group consisting of alkyl radicals of 1 to 18 carbon atoms, $-CH_2C_6H_5$, $-CH_2CH_2OH$, $-CH_2OH$ and $-(CH_2)_xNHC(O)R^{vi}$, wherein x has a value of from 2 to 10 and $R^{vi}$ is a perfluoroalkyl radical having from 1 to 12 carbon atoms;
X is chloride, bromide, fluoride, iodide, acetate or tosylate.